# EUROPEAN PATENT APPLICATION

(11) **EP 2 548 969 A1**
(43) Date of publication of application: **23.01.2013**
(21) Application number: 11756288.4
(22) Date of filing: 15.03.2011
(51) Int. Cl.: C12Q 1/02

(54) **SCREENING METHOD FOR CANDIDATE AGONIST COMPOUNDS FOR ADIPONECTIN RECEPTOR 1**

(30) Priority: 16.03.2010 US 314280 P
(71) Applicant: The University of Tokyo, Bunkyo-ku, Tokyo 1138654 (JP)
(72) Inventor: KADOWAKI, Takashi, Tokyo 113-8654 (JP); YAMAUCHI, Toshimasa, Tokyo 113-8654 (JP); IWABU, Masato, Tokyo 113-8654 (JP); IWABU, Miki, Tokyo 113-8654 (JP); TOUHARA, Kazushige, Tokyo 113-8654 (JP)
(74) Representative: Arends, William Gerrit
(86) International application number: PCT/JP2011/056039
(87) International publication number: WO 2011/115103

(57) **Abstract**

A method of screening for candidate compounds for adiponectin receptor 1 agonist including a step of bringing test compounds into contact with cells to determine whether they cause intracellular influx of extracellular calcium ions and selecting, from the test compounds, compounds causing the intracellular influx of extracellular calcium ions as the candidate compounds for adiponectin receptor 1 agonist.

SEQUENCE LISTING

<110>  The University of Tokyo
<120>  Method for screening agonist candidate compounds for adiponectin
receptor 1
<130>  111132M
<150>  U.S. Provisional Patent Application No. 61/314,280
<151>  2010-03-16
<160>  14
<170>  PatentIn version 3.1
<210>  1
<211>  23
<212>  DNA
<213>  artificial
<400>  1
acaggcacat aacatctaag gca                                              23

<210>  2
<211>  23
<212>  DNA
<213>  artificial
<400>  2
agcatgatac tgagttggcc aca                                              23

<210>  3
<211>  23
<212>  DNA
<213>  artificial
<400>  3
cggtgttgac gaggcgtccg aag                                              23

<210>  4
<211>  22
<212>  DNA
<213>  artificial
<400>  4
ggtcttcggg atgttcttcc tg                                               22

<210>  5
<211>  20
<212>  DNA
<213>  artificial
<400>  5
cgccgcataa ccagtgaaac                                                  20

<210>  6
<211>  19
<212>  DNA
<213>  artificial
<400>  6
atgtccaatt tactgaccg                                                   19

<210>  7
<211>  24
<212>  DNA
<213>  artificial
<400>  7
acagcgtgaa ttttggagtc agaa                                             24

<210>  8
<211>  20
<212>  DNA
<213>  artificial
<400>  8

gctttgctca gcctcgctat                                                    20

<210>  9
<211>  27
<212>  DNA
<213>  artificial
<400>  9
gctgaggaag atcaagcatg cccggtg                                            27

<210>  10
<211>  28
<212>  DNA
<213>  artificial
<400>  10
ttggaaaaag tccgagagac cttctctg                                           28

<210>  11
<211>  20
<212>  DNA
<213>  artificial
<400>  11
tgtccaccat cacaggaaga                                                    20

<210>  12
<211>  18
<212>  DNA
<213>  artificial
<400>  12
gcttgccctt ctcctcca                                                      18

<210>  13
<211>  23
<212>  DNA/RNA
<213>  artificial
<400>  13
gagacuggca acaucuggac att                                                23

<210>  14
<211>  23
<212>  DNA/RNA
<213>  artificial
<400>  14
gcuuagagac accuguuugu utt                                                23

## Description

### Technical Field

The present invention relates to a screening method for candidate compounds for adiponectin receptor 1 agnoist.

### Background Art

Adiponectin (J. Biol. Chem. 270, 26746-26749(1995); J. Biol. Chem. 271, 10697-10703(1996); Biochem. Biophys. Res. Commun. 221, 286-289(1996); and J. Biochem. 120, 803-812(1996)) encoded by Adipoq is adipokine having antidiabetic action and anti-atherogenic action. It has been reported (Arterioscler. Thromb. Vasc. Biol. 20, 1595-1599(2000)) that plasma adiponectin levels are decreased in obesity, insulin resistance, and type 2 diabetes. Administration of adiponectin to mice causes antihyperglycemic action and ameliorates insulin resistance (Proc. Natl Acad. Sci. USA 98, 2005-2010(2001); Nature Med. 7, 941-946(2001); and Nature Med. 7, 947-953(2001)), but adiponectin-deficient mice show insulin resistance and diabetes (J. Biol. Chem. 277, 25863-25866(2002); and Nature Med. 8, 731-737(2002)). The insulin sensitizing effect of adiponectin is presumed to appear due to an increase in fatty acid oxidation via activation of AMP-activated protein kinase (AMPK) (Nature Med. 8, 1288-1295(2002); Proc. Natl Acad. Sci. USA 99, 16309-16313(2002); and Cell Metab. 1, 15-25(2005)) and moreover via peroxisome proliferator-activated receptor α (PPARα) (Nature 405, 421-424(2000); and J. Biol. Chem. 278, 2461-2468(2003)).

There is a report (Nature 423, 762-769(2003)) on the cloning of complementary DNAs (Adipor1 and Adipor2) encoding adiponectin receptor 1 (which may hereinafter be abbreviated as "AdipoR1") and an adiponectin receptor 2 (which may hereinafter be called "AdipoR2"). AdipoR1 is much expressed in the skeletal muscle and liver, while AdipoR2 is expressed mainly in the liver. Both receptors are predicted to contain 7-transmembrane domains (Nature 423, 762-769(2003)) but to be structurally and functionally different from G-protein coupled receptors (FASEB J. 11, 346-354(1997); Nature 387, 620-624(1997); EMBO J. 15, 3566-3578(1996)). Adiponectin receptors are presumed to constitute a new receptor family.

It is indicated by using Adipor1 and/or Adipor2 knockout mice that AdipoR1 and AdipoR2 act as a main receptor for adiponectin in vivo and play an important role in the regulation of glucose and lipid metabolism, inflammation, and oxidative stress in vivo (Nature Med. 13, 332-339(2007)). In the liver, AdipoR1 activates an AMPK pathway, while AdipoR2 activates a PPARα pathway (Nature Med. 13, 332-339(2007)). On the other hand, insulin resistance has been reported to be associated with mitochondrial dysfunction (N. Engl. J. Med. 350, 664-671(2004)) but the exact cause of mitochondrial dysfunction has not yet been found. The decreased adiponectin/AdipoR1 signaling may be associated with mitochondrial dysfunction, but details of adiponectin/AdipoR1 signaling have not yet been elucidated.

Under such circumstances, there is a possibility that compounds which will be an agonist for AdipoR1 are effective for regulation of glucose and lipid metabolism, inflammation, and oxidative stress, lowering of blood sugar levels, and amelioration of insulin resistance so that they are expected to be useful as an active ingredient of pharmaceuticals. Conventionally, however, no method has been developed for efficiently screening candidate compounds for AdipoR1 agonist.

### Prior Art Documents

### Non-patent Documents

Non-patent Document 1: J. Biol. Chem. 270, 26746-26749(1995)
Non-patent Document 2: J. Biol. Chem. 271, 10697-10703(1996)
Non-patent Document 3: Biochem. Biophys. Res. Commun. 221, 286-289(1996)
Non-patent Document 4: J. Biochem. 120, 803-812(1996)
Non-patent Document 5: Arterioscler. Thromb. Vasc. Biol. 20, 1595-1599(2000)
Non-patent Document 6: Proc. Natl Acad. Sci. USA 98, 2005-2010(2001)
Non-patent Document 7: Nature Med. 7, 941-946(2001)
Non-patent Document 8: Nature Med. 7, 947-953(2001)
Non-patent Document 9: J. Biol. Chem. 277, 25863-25866(2002)
Non-patent Document 10: Nature Med. 8, 731-737(2002)
Non-patent Document 11: Nature Med. 8, 1288-1295(2002)
Non-patent Document 12: Proc. Natl Acad. Sci. USA 99, 16309-16313(2002)
Non-patent Document 13: Cell Metab. 1, 15-25(2005)
Non-patent Document 14: Nature 405, 421-424(2000)
Non-patent Document 15: J. Biol. Chem. 278, 2461-2468(2003)
Non-patent Document 16: Nature 423, 762-769(2003)
Non-patent Document 17: Nature 423, 762-769(2003)
Non-patent Document 18: FASEB J. 11, 346-354(1997)
Non-patent Document 19: Nature 387, 620-624(1997)
Non-patent Document 20: EMBO J. 15, 3566-3578(1996)
Non-patent Document 21: Nature Med. 13, 332-339(2007)
Non-patent Document 22: Nature Med. 13, 332-339(2007)
Non-patent Document 23: N. Engl. J. Med. 350, 664-671(2004)

### Summary of the Invention

### Problem to be Solved by the Invention

An object of the present invention is to provide a screening method for candidate compounds for adiponectin receptor 1 agonist.

### Means for Solving the Problem

The present inventors have carried out an extensive investigation with a view to solving the above-mentioned problem. As a result, it has been found that adiponectin bound to adiponectin receptor 1 causes incorporation of calcium ions (Ca²⁺) in skeletal muscle cells. It has also been found that influx, into skeletal muscle cells, of calcium ions generated when adiponectin binds to AdipoR1 is indispensable for subsequent activation of calcium ion/calmodulin-dependent protein kinase kinase β (CaMKKβ), activation of AMPK and SIRT1, increased expression and decreased acetylation of peroxisome proliferator-activated receptor γ coactivator-1 α (PGC-1α), and increased mitochondria in the muscle cells. It has also been confirmed, on the other hand, that muscle-specific disruption of adiponectin receptor 1 results in decreased elevation of a calcium ion concentration by adiponectin in cells which leads to decreased activation of CaMKK, AMPK, and SIRT1 and suppression of AdipoR1 results in decreased expression and deacetylation of PGC-1α, a decreased mitochondrial content and enzymes, decreased oxidative type I myofibers and decreased oxidative stress-detoxifying enzymes in skeletal muscle.

The present inventors have therefore revealed that various physiological activities induced by binding of adiponectin to adiponectin receptor 1 are induced by incorporation of calcium ions in skeletal muscle cells which occurs first by binding of adiponectin to adiponectin receptor 1. Based on this finding, it has been found that compounds having an agonist action for adiponectin receptor 1 can be screened using incorporation of calcium ions in skeletal muscle cells as an indicator. It has also been found that it is possible to employ, as the screening method, a method of bringing a test compound into contact with skeletal muscle cells and then measuring the incorporation of calcium ions, for example, by detecting electrical signals or a method of respectively bringing a test compound into AdipoRl-deficieint cells and AdipoR1 expressing cells and measuring incorporation of calcium ions, for example, by detecting electrical signals; and to achieve high-speed and high-accuracy screening by using these methods in combination as first screening and second screening. The present invention has been completed based on these findings.

Described specifically, the present invention provides a method of screening for candidate compounds for adiponectin receptor 1 agonist, including a step of bringing test compounds into contact with cells to determine whether or not the test compounds cause intracellular influx of extracellular calcium ions; and selecting, from the test compounds, compounds causing intracellular influx of extracellular calcium ions as the candidate compounds for adiponectin receptor 1 agonist.

According to preferred modes of the above-mentioned invention, provided are the above-mentioned method wherein the cells are skeletal muscle cells; the above-mentioned method wherein the cells are *Xenopus* oocytes injected with AdipoR1 complementary RNA; the above-mentioned method further including a step of measuring an intracellular calcium ion concentration; the above-mentioned method wherein the intracellular calcium concentration is measured in terms of fluorescence intensity by using a calcium ion fluorescent reagent; the above-mentioned method wherein the intracellular calcium ion concentration is measured electrophysiologically; the above-mentioned method wherein as the cells, cells in which adiponectin receptor 1 has been expressed are used; and the above-mentioned method wherein the cells in which adiponectin receptor 1 has been expressed are oocytes.

According to a more preferred mode, provided is a method of bringing test compounds into contact with AdipoR1-deficient cells and AdipoR1 expressing cells to determine whether or not the test compounds cause intracellular influx of extracellular calcium ions and selecting, from the test compounds, compounds not substantially causing intracellular influx of extracellular calcium ions in the AdipoR1-deficient cells but causing intracellular influx of extracellular calcium ions in the AdipoR1 expressing cells as the candidate compounds for adiponectin receptor 1 agonist.

Further, provided is a method of screening for candidate compounds for adiponectin receptor 1 agonist including the following steps:
(a) a step of bringing test compounds into contact with cells to determine whether or not the test compounds cause intracellular influx of extracellular calcium ions and selecting, from the test compounds, compounds causing intracellular influx of extracellular calcium ions as the candidate compounds for adiponectin receptor 1 agonist; and
(b) bringing the candidate agonist compounds selected in the step (a) into contact with AdipoR1-deficient cells and AdipoR1 expressing cells to determine whether the candidate agonist compounds cause intracellular influx of extracellular calcium ions or not and selecting, from the candidate agonist compounds, compounds not causing intracellular influx of extracellular calcium ions in the AdipoR1-deficient cells but causing intracellular influx of extracellular calcium ions in the AdipoR1 expressing cells as adiponectin receptor 1 agonist compounds.

According to preferred modes of the above-described present invention, provided are the above-mentioned method wherein the cells are skeletal muscle cells; the above-mentioned method further including a step of measuring an intracellular calcium ion concentration; and the above-mentioned method wherein the intracellular calcium ion concentration is measured in terms of fluorescence intensity by using a calcium ion fluorescent reagent.

### Effect of the Invention

By using the method of the present invention, candidate compounds for adiponectin receptor 1 agonist can be screened very easily and precisely. The adiponectin receptor 1 agonist compounds are expected as an active ingredient of a pharmaceutical for treating and/or preventing obesity or treating and/or preventing diabetes. In addition, as described specifically in detail in Examples herein, since adiponectin receptor 1 bound to adiponectin causes influx of extracellular calcium ions into skeletal muscle cells and is thereby involved in regulation of calcium ion signaling, regulation of glucose and lipid metabolism, regulation of exercise endurance, regulation of expression and activation of PGC-1α through AMPK/SIRT1, and regulation of mitochondrial function and oxidative stress, adiponectin receptor 1 agonist compounds bound to adiponectin receptor 1 cause similar physiological actions and are therefore expected to be useful as an active ingredient of various pharmaceuticals.

For example, these physiological changes induced in skeletal muscle cells after influx of calcium ions are substantially similar to post-exercise changes in skeletal muscle cells so that adiponectin receptor 1 agonist compounds can be used as an active ingredient of pharmaceuticals capable of producing a pseudo-exercise effect. For example, for diabetic patients who are of advanced age or have difficulty in walking and therefore cannot be subjected to exercise therapy, such pharmaceuticals can be used instead of exercise therapy or as an auxiliary pharmaceutical therefor.

### Brief Description of the Drawings

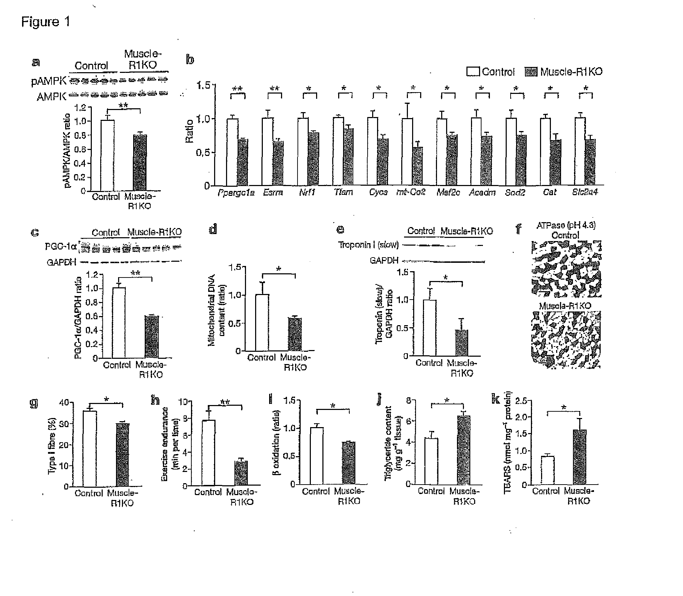

[FIG. 1] FIG. 1 shows a decreased mitochondrial content and the number of oxidative type I myofibers and reduced exercise capacity in skeletal muscle of muscle-R1KO mice. FIGS. (a) to (k) show results of skeletal muscle (a-g, i-k) obtained from Control or muscle-R1 knockout (KO) mice after 5-hour fasting, in which (a) shows phosphorylation and amount of AMPK, (b) mRNA level of Ppargcla, Esrra, Nrf1, Tfam, Cycs, mt-Co2, Mef2c, Acadm, Sod2, Cat, and Slc2a4, (c)PGC-1α protein level, (d) a mitochondrial content as assessed by the mitochondrial DNA copy number, (e) amounts of troponin I (slow) protein, (f) soleus muscles analyzed by ATPase (pH 4.3 for type I fibers) staining (Scale bars, 100 µm), (g) quantification of fiber type distribution (f) based on fiber-type analyses, (h) exercise endurance, (i) β oxidation, (j) a triglyceride content, and (k) TBARS. All values were presented as mean ± s.e.m (n=5-12). Symbols * and ** mean P<0.05 and P<0.01, respectively, compared with control mice.

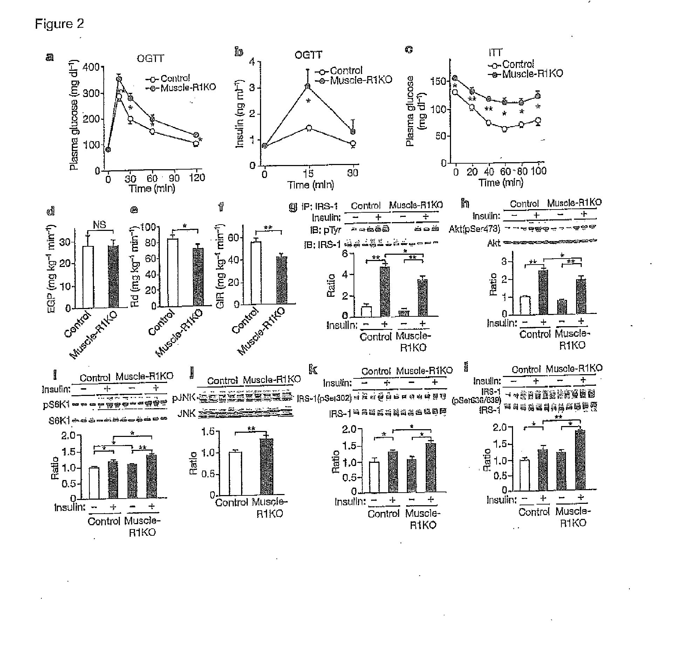

[FIG. 2] FIG. 2 shows mechanisms of abnormal glucose and insulin homeostasis in muscle-R1KO mice. FIGS. (a) to (f) show results of hyperinsulinemic euglycermic clamp study in control and muscle-R1KO mice, more specifically, (a,c) plasma glucose and (b) plasma insulin during (a,b) oral glucose tolerance test (OGTT) (1.5 g glucose per kg body weight) or (c) during insulin tolerance test (ITT) (0.25U insulin per kg body weight); (d) endogenous glucose production (EGP), (e) rates of glucose disposal (Rd), and (f) glucose infusion rate (GIR). FIGS. (g) to (l) show (g) phosphorylation of tyrosine (pTyr) in IRS-1, (k) phosphorylation of Ser 302 in IRS-1, (l) phosphorylation of Ser 636/639 in IRS-1, (h) phosphorylation and amount of Akt, (i) phosphorylation and amount of S6K1, and (j) phosphorylation and amount of JNK in skeletal muscle treated with or without insulin (0.3 U per kg body weight) for 7.5 minutes in control mice and muscle R1KO mice after 5-hr fasting. IB means immunoblotting, while IP means immunoprecipitation. All values were presented as mean ± s.e.m. Symbols * and ** mean, in from 3 to 5 independent experiments (n= 6 to 15), P<0.05 and P<0.01, respectively, compared with control or indicated one and NS means no significant difference.

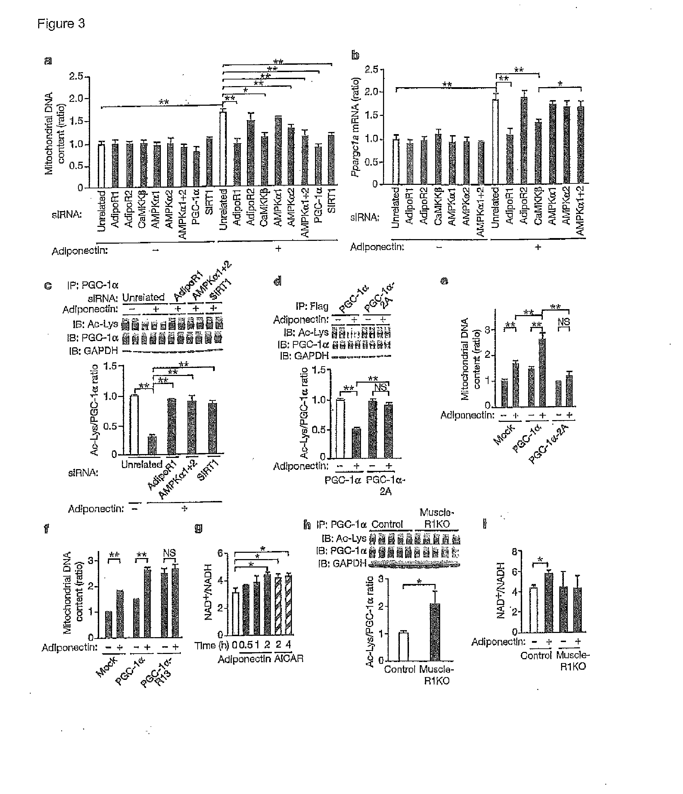

[FIG. 3] FIG. 3 shows the result of PGC-1α expression and activation and mitochondrial biogenesis, in C2C12 myocytes, increased by adiponectin/AdipoR1. Shown in (a) to (i) are mitochondrial contents as assessed by the mitochondrial DNA copy number (a, e, f), Ppargcla mRNA levels (b), acetyl-lysine (Ac-Lys) levels or flag immunoprecipitates (IP) checked on PGC-1α (c, d, h), and an NAD⁺/NADH ratio (g, i) in C2C12 myocytes treated with adiponectin for indicated time (g); in C2C12 myocytes (a to c) treated with 10 µgml⁻¹ adiponectin for 48 hours (a, e, f) or for 1.5 hours (b) or 2 hours (c, d) and then transfected with the indicated siRNA duplex (a to c), with the wild-type or the 2A mutant form of PGC-1α (d, e), or with the R13 mutant form of PGC-1α (f); or in skeletal muscles (h, i) from control mice or muscle-R1KO mice treated with or without adiponectin. The supernatant was blotted against GAPDH as input control (c, d, h). C2C12 myocytes were used after myogenic differentiation in all experiments. All values were presented as mean ± s.e.m (n=5-10). Symbols * and ** mean P<0.05 and P<0.01, respectively, compared with control or unrelated siRNA or as indicated.

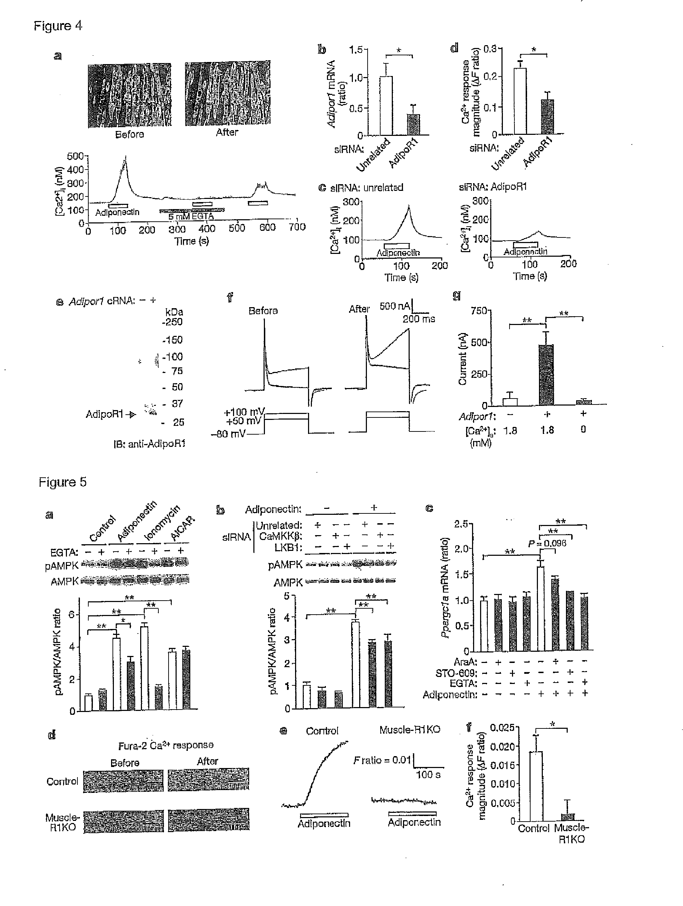

[FIG. 4] FIG. 4 shows adiponectin-induced calcium ion influx via AdipoR1 in C2C12 myocytes and *Xenopus* oocytes. FIG. (a) shows images of changes in fura-2 1 minute before and after stimulation with adiponectin (30 µgml⁻¹). The red portion corresponds to the greatest response. A bottom trace demonstrated the average calcium response of C2C12 myocytes to 1-min stimulation with adiponectin, while including application of 5mM EGTA (black bar). The shaded region around the trace represents s.e.m. (b) to (d): FIG. (b) shows Adipor1 mRNA levels (b), fura-2 calcium response (c), and the magnitude of the response (d) in C2C12 myocytes transfected with unrelated siRNA duplex or AdipoR1 siRNA duplex in response to stimulation with 30 µgml⁻¹ adiponectin for 1 min. (e) to (g): FIG. (e) shows the amounts of AdipoR1 protein in *Xenopus* oocytes (depolarizing pulses of +100 mV) injected with or without Adipor1 cRNA corresponding to adiponectin (30 µg/ml), or added with or without application of 5 mM EGTA, FIG. (f) shows representative traces of calcium-ion-activated C1⁻ current 30 seconds before (left) and after (right) administration of adiponectin, and FIG. (g) shows the magnitude of them. [Calcium ion]ᵢ and [calcium ion]ₑ mean intracellular and extracellular calcium ion concentrations, respectively. All values were presented as mean ± s.e.m (n=6-14). Symbols * and ** mean P<0.05 and P<0.01, respectively, compared with unrelated siRNA cells or control cells or as indicated.
[FIG. 5] FIG. 5 shows the necessity of adiponectin-induced calcium ion influx for CaMKK and AMPK activation and PGC-1α expression. (a) and (b): FIG. (a) shows phosphorylation and amount of AMPK in C2C12 myocytes preincubated for 20 min with or without 5mM EGTA and then, treated for 5 min with adiponectin (30 µgml⁻¹) or ionomycin (1 µM), or for 1 hr with AICAR(1 mM) and FIG. (b) shows phosphorylation and amount of AMPK in C2C12 myocytes transfected with the indicated siRNA duplex and then treated with 30 µgml⁻¹ adiponectin for 5 min. FIG. (c) shows the amount of Ppargcla mRNA in C2C12 myocytes preincubated for 1 hr with AraA (0.5 mM), for 6 hr with STO-609 (1 µgml⁻¹), or for 20 min with EGTA (5 mM) and then treated for 1.5 hr with or without adiponectin (10 µgml⁻¹). FIG. (d) shows representative images of changes in fura-2 calcium response 5 min before or after stimulation with adiponectin (30 µgml⁻¹) in a soleus muscle from control mice (top) and muscle-R1KO mice (bottom). The red portion corresponds to the greatest response and scale bars indicate 100 µm. FIG. (e) shows a trace demonstrates the calcium response of a soleus muscle in the fields presented in (d). Adiponectin was administered for the time as indicated. FIG. (f) shows the magnitude of a fura-2 calcium response signal caused by 160-sec adiponectin stimulation to a soleus muscle and a ΔF ratio shows a change in fluorescence ratio after administration of adiponectin. All values were presented as mean ± s.e.m (n=5-10). Symbols * and ** mean P<0.05 and P<0.01, respectively, compared with control cells or unrelated siRNA cells or control cells or as indicated.

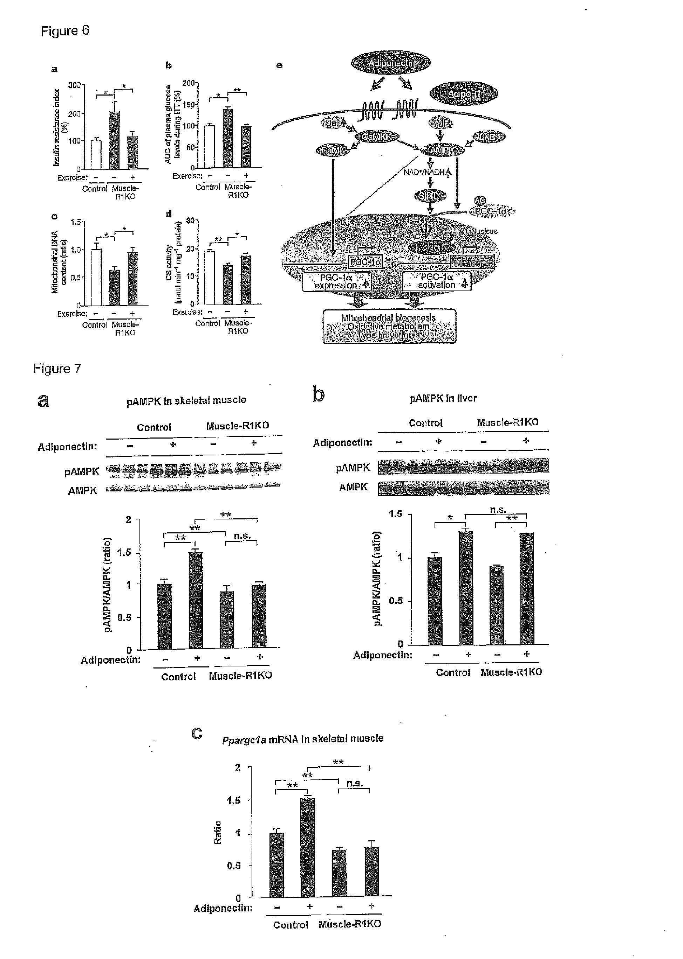

[FIG. 6] FIG. 6 shows the effect of exercise on muscle-R1KO mice. FIGS. (a) to (d) show an insulin resistance index in skeletal muscles (a), an area under the blood level curves (AUC) of plasma glucose levels during ITT (b), a mitochondrial content as assessed by the mitochondrial DNA copy number (c), and citrate synthase (CS) enzyme activity (d) of control and muscle-R1KO mice after 2-week exercise. The results are expressed as the percentage of the value in control littermates (a, b). FIG. (e) shows a scheme illustrating the signaling of adiponectin/AdipoR1 in myocytes. Both CaMKKβ and LKB1 are necessary for adiponectin-induced full AMPK activation. AMPK and SIRT1 are required for adiponectin/AdipoR1-induced PGC-1α activation. CaMKKβ activation by adiponectin-induced calcium ion influx via AdipoR1 is required for adiponectin-induced increased PGC-1α expression. PGC-1α is required for mitochondrial biogenesis stimulated with adiponectin/AdipoR1. From these data, the present inventors concluded that adiponectin and AdipoR1 increase PGC-1α expression and activity via calcium ion signaling and AMPK/SIRT1, leading to increased mitochondrial biogenesis. The present inventors focused to the molecules on which they have obtained direct evidence by both gain-of-function and loss-of-function experiments in vitro and in vivo (except for CaMK which has already been reported (Nature 454, 463-469(2008) to increase PGC-1α expression by other researches). AC means acetylation. All values were presented as mean ± s.e.m (n=5-8). Symbols * and ** mean P<0.05 and P<0.01, respectively, compared with control mice or as indicated.
[FIG. 7] FIG. 7 shows that muscle-specific disruption of AdipoR1 results in decrease of adiponectin action in skeletal muscle. (a), (b): Phosphorylation and amount of AMPK in skeletal muscle (a) and liver (b) treated with or without adiponectin (30 µg per 10 g body weight) for 10 min. (c): PGC-1α (Ppargcla) mRNA levels in skeletal muscle treated with or without adiponectin (30 µg per 10 g body weight) for 10 min. All values are presented as mean ± s.e.m (n=5-8). Symbols * and ** mean P<0.05 and P<0.01, respectively, compared with control mice.

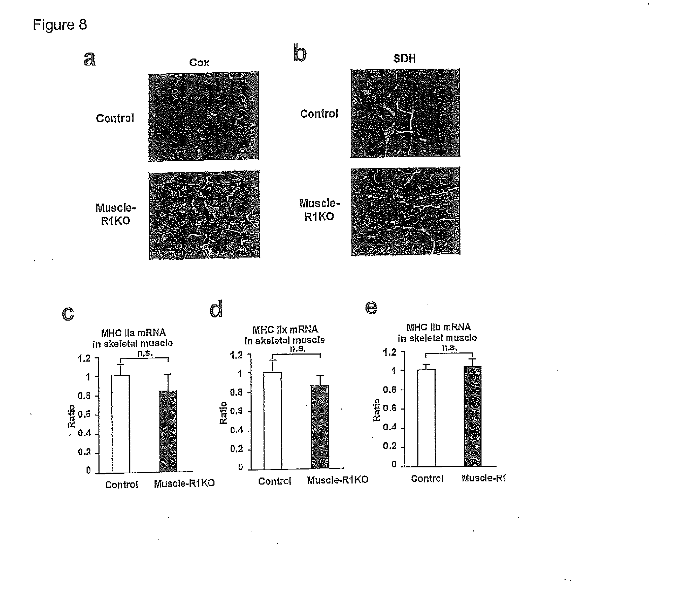

[FIG. 8] FIG. 8 shows a decreased mitochondrial content, function, type II fiber in skeletal muscle of muscle-R1KO mice. (a) and (b): The cross-sections of soleus muscle were stained for Cox activity (a) and SDH activity (b). Scale bars: 100 µm. Skeletal muscle was obtained from control mice or muscle-R1KO mice. (c) to (e): MHC IIa (c), MHC IIx (d), and MHC IIb (e) mRNA levels in skeletal muscle were analyzed by RT-qPCR. All values were presented as mean ± s.e.m (n=5-8). Symbols * and ** mean P<0.05 and P<0.01, respectively, compared with control mice.

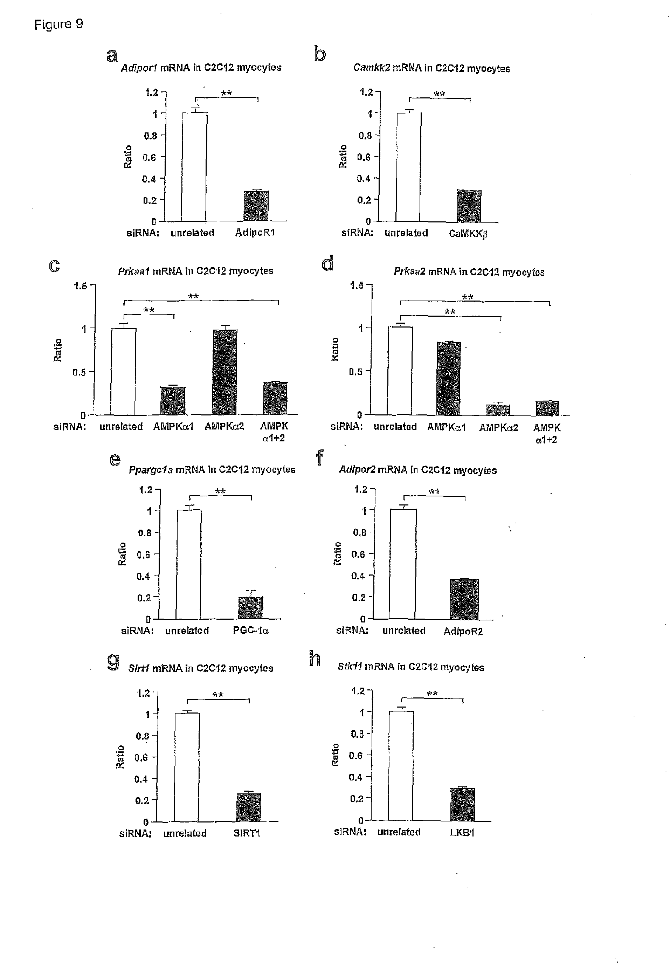

[FIG. 9] FIG. 9 shows expression of mRNA in C2C12 cells transfected with siRNA duplex. (a) to (h): C2C12 myocytes were transfected with siRNA duplex. AdipoR1 (Adipor1) (a), CaMKKβ (Camkk2) (b), AMPKα1 (Prkaa1) (c), AMPKα2 (Prkaa2) (d), PGC-1α (Ppargcla) (e), AdipoR2 (Adipor2) (f), SIRT1 (Sirt1) (g), and LKB1 (Stk11) (h) mRNA levels were analyzed by RT-qPCR. All values were presented as mean ± s.e.m (n=8-10). Symbol ** means P<0.01 compared with unrelated siRNA cells.

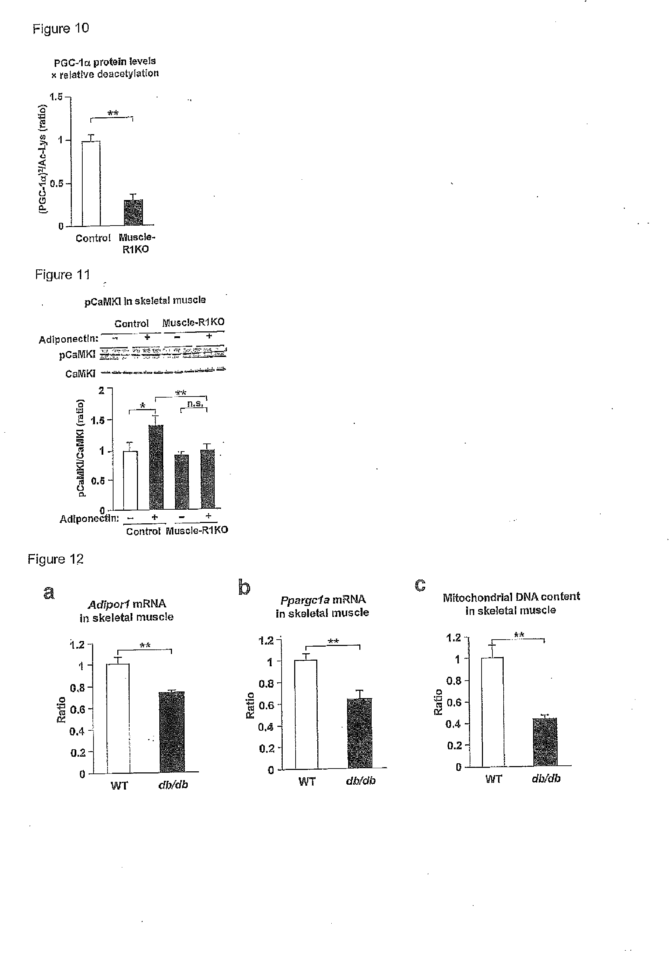

[FIG. 10] FIG. 10 shows total activity of PGC-1α in skeletal muscle. It shows total activity as assessed by multiplying expression and relative deacetylation of PGC-1α together. Skeletal muscle was obtained from control mice or muscle-R1KO mice. All values were presented as mean ± s.e.m (n=8-10). Symbols * and ** mean P<0.05 and P<0.01, respectively, compared to control or as indicated.
[FIG. 11] FIG. 11 shows phosphorylation of CaMKI stimulated with adiponectin in skeletal muscle. It shows phosphorylation and amount of CaMKI in the skeletal muscle of control and muscle-R1KO mice treated for 5 min with or without adiponectin (30 µg per 10 g body weight). All values were presented as mean ± s.e.m (n=3-6). Symbols * and ** mean P<0.05 and P<0.01, respectively, compared with control or as indicated.
[FIG. 12] FIG. 12 shows a decreased mitochondrial content in skeletal muscle of db/db mice. (a) and (b): Adipor1 (a) and Ppargcla (b) mRNA levels in the skeletal muscle of wild-type (WT) and db/db mice were analyzed by RT-qPCR. (c): a mitochondrial content as assessed by the mitochondrial DNA copy number. Skeletal muscle was obtained from WT mice or db/db mice. All values were presented as mean ± s.e.m (n=5-10). Symbol ** means P<0.01 compared with WT mice.

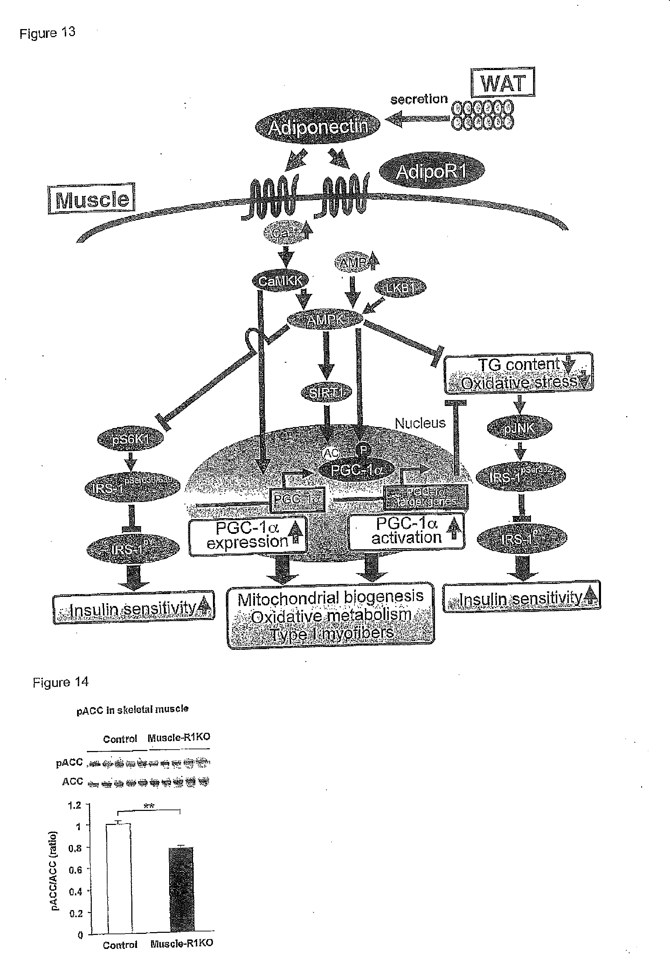

[FIG. 13] FIG. 13 is a scheme illustrating the actions of adiponectin/AdipoR1 in skeletal muscle. Adiponectin/AdipoR1 increased phosphorylation of AMPK, the amount and activity of PGC-1α, mitochondrial biogenesis, type I myofibers, and insulin sensitivity.
[FIG. 14] FIG. 14 shows that muscle-specific disruption of AdipoR1 results in decrease of adiponectin action in skeletal muscle. Phosphorylation and amount of ACC in the skeletal muscle of muscle-R1KO mice. All values were presented as mean ± s.e.m (n=5). Symbol ** means P<0.01 compared with control mice.

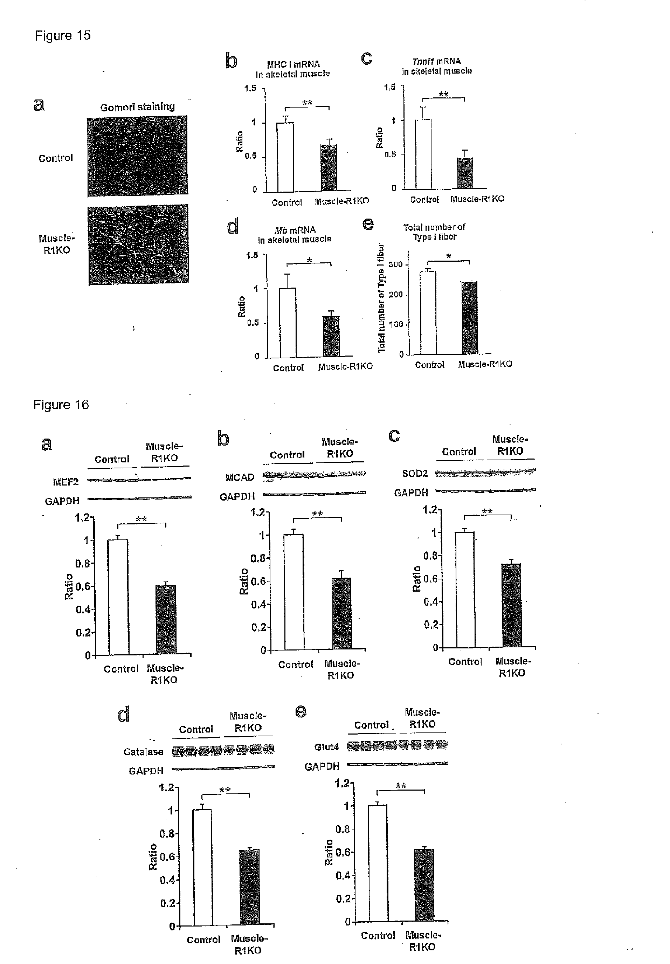

[FIG. 15] FIG. 15 shows a decreased mitochondrial content and type I fibers in the skeletal muscle of muscle-R1KO mice. (a): Modified Gomori trichrome staining. Scale bars: 100 µm. Skeletal muscle was obtained from control mice or muscle-R1KO mice. (b) to (e): MHC I (b), troponin I (slow) (Tnni1) (c), and myoglobin (Mb) (d) mRNA levels in skeletal muscle were analyzed by RT-qPCR. (e): Quantification of fiber type distribution based on the fiber type analysis in FIG. 1f. All values were presented as mean ± s.e.m (n=5-8). Symbols * and ** mean P<0.05 and P<0.01, respectively, compared with control.
[FIG. 16] FIG. 16 shows decreased expression levels of proteins important for the skeletal muscle in muscle-R1KO mice. (a) to (e): MEF2 (a), MCAD (b), SOD2 (c), catalase (d), and Glut4 (e) proteins in the skeletal muscle. The skeletal muscle was obtained from control mice or muscle-R1KO mice. All values were presented as mean ± s.e.m (n=4-8). Symbol ** means P<0.01, compared with control mice.

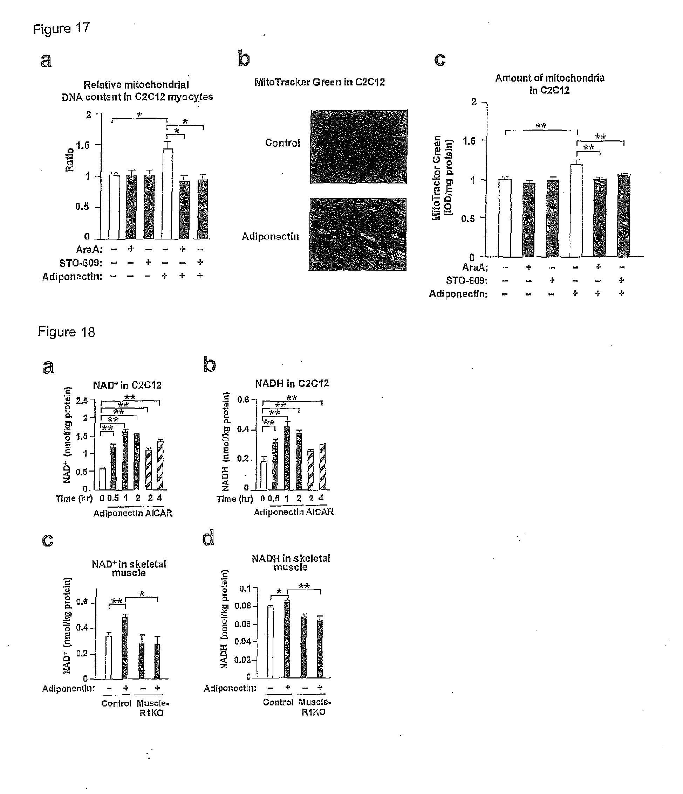

[FIG. 17] FIG. 17 shows an increased amount of mitochondria in C2C12 myocytes treated with adiponectin. (a): A mitochondrial content as assessed by the mitochondrial DNA copy number in C2C12 myocytes preincubated with AraA (0.5 mM) for 1 hr or with STO-609 (1 µg/ml) for 6 hr and then treated with 10 µg/ml adiponectin for 48 hr. (b): After treatment of C2C12 myocytes with 10 µg/ml adiponectin for 48 hr, 100 nm MitoTracker green FM was added and the resulting mixture was incubated for 30 min. (c): An amount of mitochondria was indicated by green fluorescence and a mitochondrial content was measured by using MitoTracker green. All values were presented as mean ± s.e.m (n=5-8). Symbol ** means P<0.01 compared as indicated.
[FIG. 18] FIG. 18 shows NAD⁺ and NADH contents in C2C12 myocytes and skeletal muscle. (a) to (d): NAD⁺ (a, c) and NADH (b, d) contents in C2C12 myocytes (a, b) and skeletal muscles (c, d) treated with adiponectin for the times indicated. Skeletal muscle was obtained from control mice or muscle-R1KO mice. All values were presented as mean ± s.e.m (n=8-10). Symbols * and ** mean P<0.05 and P<0.01, respectively, compared with control or as indicated.

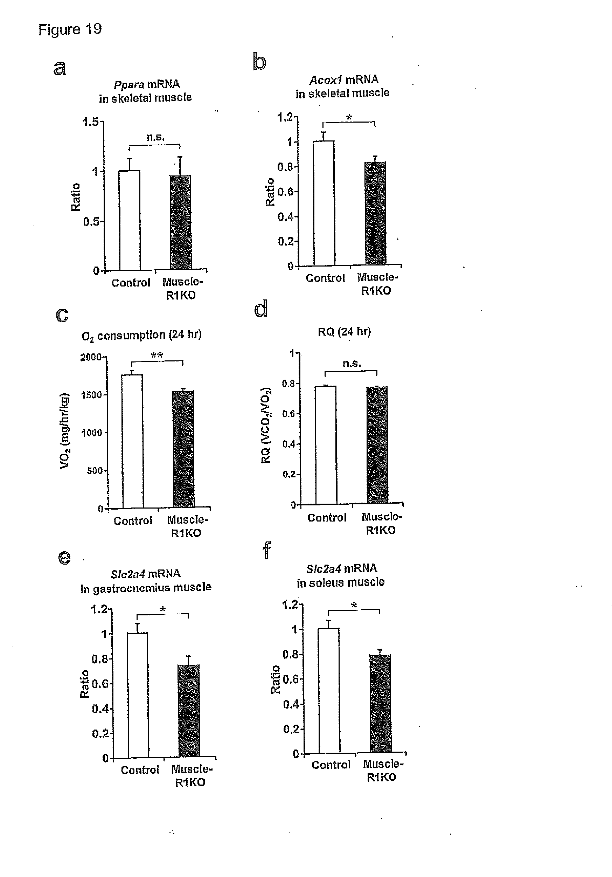

[FIG. 19] FIG. 19 shows Pparα, Aco, and Slc2a4 mRNA levels, oxygen (O₂) consumption, and respiratory quotient (RQ) in the skeletal muscle of muscle-R1KO mice. (a) and (b): PPARα (Ppara) (a) and Aco (Acox1)(b) mRNA levels in the skeletal muscle of control mice and muscle-R1KO mice were analyzed by RT-qPCR. (c) and (d): Mean 24-hr values of oxygen (O₂) consumption (c) and respiratory quotient (RQ) (d) in control mice and muscle-R1KO mice. (e) and (f): Glut4 (Slc2a4) mRNA levels in the gastrocnemius muscle (e) and soleus muscle (f) of control mice and muscle-R1KO mice were analyzed by RT-qPCR. All values were presented as mean ± s.e.m (n=6-14). Symbols * and ** mean P<0.05 and P<0.01, respectively, compared with control mice.

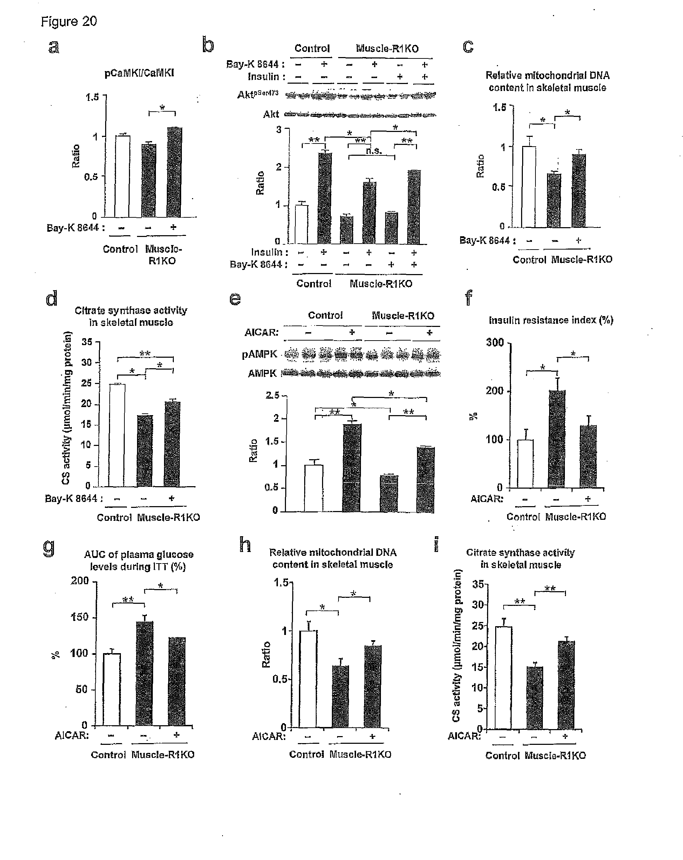

[FIG. 20] FIG. 20 shows the effect of increased calcium influx and AICAR in the muscle of muscle-R1KO mice. (a) to (d): Control mice and muscle-R1KO mice were treated with or without 0.25 µM Bay-K 8644 as a calcium-channel opener. (f) to (i): Control mice and muscle-R1KO mice were treated with a solvent or AICAR (500 mg per kg body weight per day, 2 weeks). (a): The relative ratio of phosphorylation of CaMKI in the skeletal muscle of muscle-R1KO mice treated with or without 0.25 µM Bay-K 8644. (b): Phosphorylation and amount of Akt (Ser 473) in skeletal muscle treated with or without 0.25 µM Bay-K 8644. (c) and (h): Mitochondrial content in skeletal muscle as assessed by the mitochondrial DNA copy number. Skeletal muscle was obtained from muscle-R1KO mice. (d) and (i): Citrate synthase enzyme activity in the skeletal muscle of control mice and Muscle-R1KO mice. (e): Phosphorylation and amount of AMPK in skeletal muscle treated with or without AICAR (500 mg per kg body weight per day, 2 hours). (f) Insulin resistance index. The insulin resistance index was calculated from the product of the areas of glucose and insulin x 10⁻² in the glucose tolerance test. The results were expressed as the percentage of the value of control littermates. (g): Area under the curves (AUC) of plasma glucose levels during the insulin tolerance test (ITT). All values were presented as mean ± s.e.m (n=5-8). Symbols * and ** mean P<0.05 and P<0.01, respectively, compared with control mice.

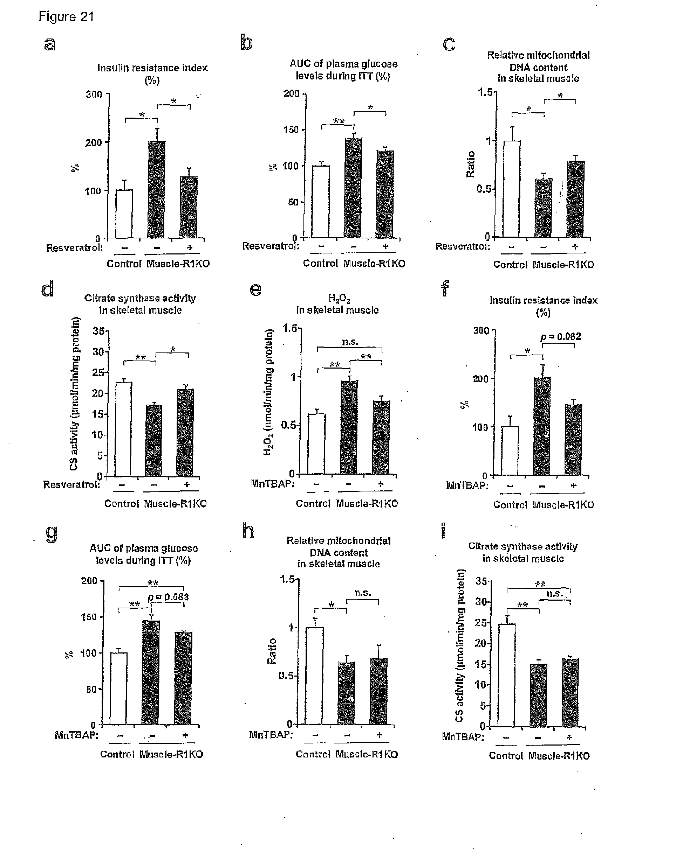

[FIG. 21] FIG. 21 shows the effect of resveratrol and antioxidant MnTBAP on muscle-R1KO mice. (a) to (d): Control mice and muscle-R1KO mice were treated with a solvent or resveratrol (400 mg per kg body weight per day, 2 weeks). (e) to (i): Control mice and muscle-R1KO mice were treated with a solvent or an antioxidant MnTBAP (10 mg per kg body weight per day, 2 weeks). (a) and (f): Insulin resistance index. The insulin resistance index was calculated from the product of the areas of glucose and insulin x 10⁻² in the glucose tolerance test. The results were expressed as the percentage of the value of control littermates. (b) and (g): Area under the curves (AUC) of plasma glucose levels during the insulin tolerance test (ITT). (c) and (h): Mitochondrial content in skeletal muscle as assessed by the mitochondrial DNA copy number. The skeletal muscle was obtained from control mice or muscle-R1KO mice. (d) and (i): Citrate synthase enzyme activity in the skeletal muscle of control mice and muscle-R1KO mice. (e): The production of reactive oxygen species (ROS) as H₂O₂ in the skeletal muscle of control mice and muscle-R1KO mice treated with a solvent or an antioxidant MnTBAP (10 mg per kg body weight per day, 2 weeks). All values were presented as mean ± s.e.m (n=5-8). Symbols * and ** mean P<0.05 and P<0.01, respectively, compared with control mice or as indicated.

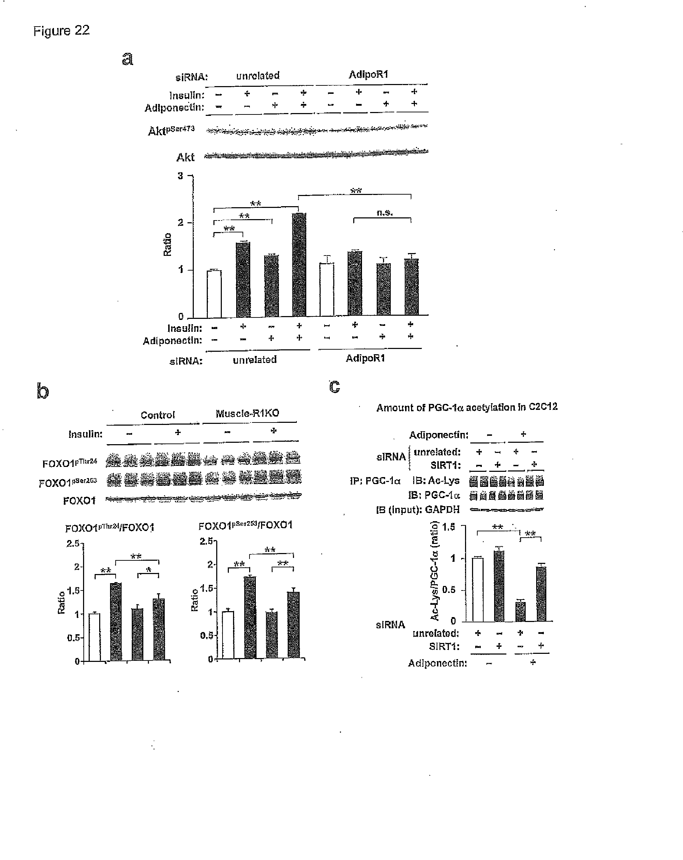

[FIG. 22] FIG. 22 shows insulin-induced phosphorylation of Akt in C2C12 myocytes, FOXO1 phosphorylation in skeletal muscle, and an amount of PGC-1α acetylation in C2C12 myocytes. (a): Phosphorylation (Ser 473) and amount of Akt in C2C12 myocytes treated with or without 10 µg/ml adiponectin for 48 hr and then treated with or without 10 nM insulin for 10 min. (b) Phosphorylation (Thr 24 and Ser 253) and amount of FOXO1 in the skeletal muscle of muscle-R1KO mice treated with or without insulin (0.3 U per kg body weight) for 7.5 min. (c): C2C12 myocytes were transfected with SIRT1 siRNA duplex and then treated for 2 hr with or without adiponectin (10 µg/ml). Acetyl-lysine levels were checked on PGC-1α immunoprecipitates (IP). The supernatant was blotted against GAPDH as input control. All values were presented as mean + s.e.m (n=6-10). Symbols * and ** mean P<0.05 and P<0.01, respectively, compared with control or as indicated.

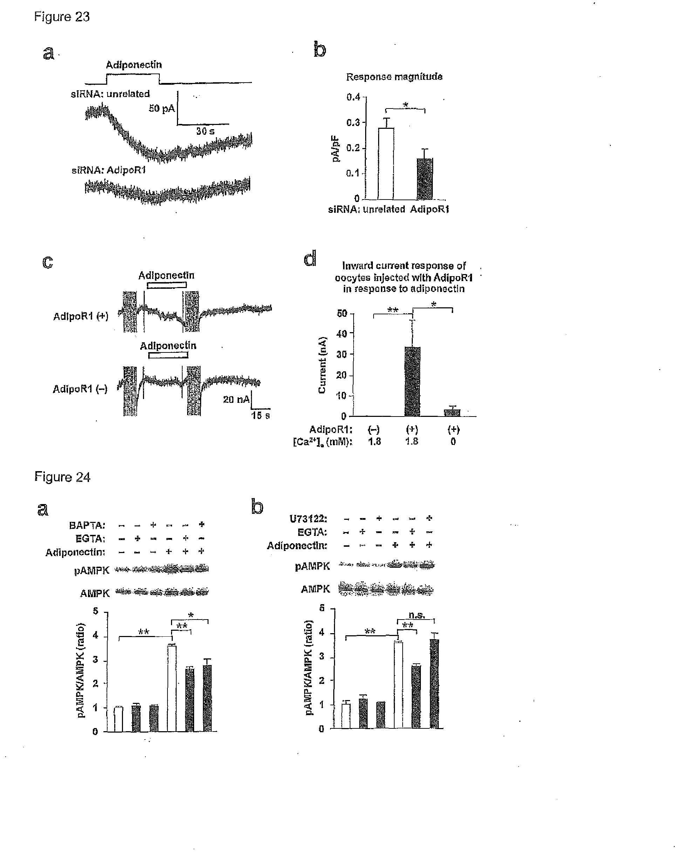

[FIG. 23] FIG. 23 shows adiponectin-induced inward current response via AdipoR1 in C2C12 myocytes and *Xenopus* oocytes. (a): The whole-cell inward current response of C2C12 myocytes, which were transfected with an unrelated siRNA duplex (upper trace) and an AdipoR1 siRNA duplex (bottom trace), to stimulation with 30 µg/ml adiponectin for 30 sec. Holding potential was -60 mV. (b): Effects of AdipoR1 knockdown on the whole-cell inward current response to adiponectin. (c): Representative current traces of oocytes injected with (top) or without (bottom) AdipoR1 cRNA in response to adiponectin recorded at a holding potential of -80 mV. Adiponectin (30 µg/ml) was administered for 30 sec at the times indicated by squares. (d): The magnitude of inward current response of oocytes injected with or without AdipoR1 cRNA in response to adiponectin (30 µg/ml) along with or without administration of 5 mM EGTA. [Calcium ion]₍ₑ₎: external calcium ion concentration. All values were presented as mean ± s.e.m (n=6-14). Symbols * and ** mean P<0.05 and P<0.01, respectively, compared with the unrelated siRNA cells or as indicated.
[FIG. 24] FIG. 24 shows phosphorylation of AMPK stimulated with adiponectin in the presence of BAPTA-AM or U73122. (a) and (b): Phosphorylation and amount of AMPK in C2C12 myocytes. C2C12 myocytes were preincubated for 30 min after addition of 50 µM BAPTA-AM or for 20 min after addition of 5 mM EGTA and then treated for 5 min with adiponectin (30 µg/ml) (a). C2C12 myocytes were preincubated for 30 min after addition of 10 µM U73122 or for 20 min after addition of 5 mM EGTA and treated for 5 min with adiponectin (30 µg/ml) (b). All values were presented as mean ± s.e.m (n=3-6). Symbols * and ** mean P<0.05 and P<0.01, respectively, compared with control or as indicated.

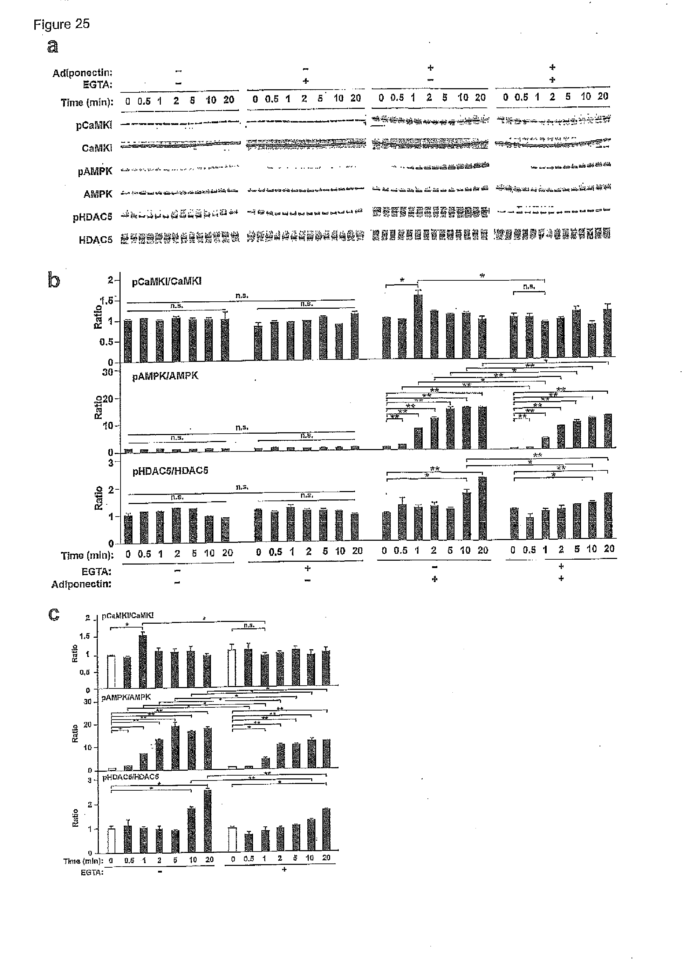

[FIG. 25] FIG. 25 shows phosphorylation of CaMKI, AMPK, and HDAC5 stimulated with adiponectin in the presence of EGTA. (a): Phosphorylation and amount of CaMKI, AMPK, and HDAC5 in C2C12 myocytes. C2C12 myocytes were preincubated for 20 min with or without EGTA (5 mM) and then treated for 0, 0.5, 1, 2, 5, 10 and 20 min with or without EGTA (5 mM) and with or without adiponectin (30 µg/ml). (b): Densitometric quantification of immunoblots (a) of phosphorylation and amount of CaMKI (upper panel), AMPK (middle panel), and HDAC5 (lower panel) levels. (c) Densitometric quantification of immunoblots (a) of phorphorylation and amount of CaMKI (upper panel), AMPK (middle panel), and HDAC5 (lower panel) levels. The result of each time point is corrected for any change in the basal status during incubation in calcium-ion-free media without adiponectin (matched controls). All values were presented as mean ± s.e.m (n=3-8). Symbols * and ** mean P<0.05 and P<0.01, respectively, compared with control or as indicated.

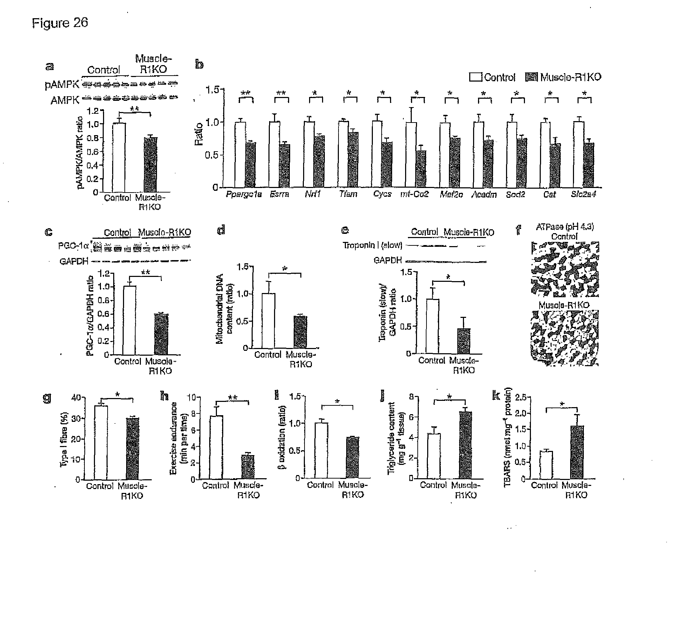

[FIG. 26] FIG. 26 shows generation and characterization of muscle-R1KO mice. (a): Schematic view of the Adipor1 gene targeting strategy. Muscle-R1KO mice were generated by crossing mice with a floxed Adipor1 allele to animals that transgenically express cre recombinase under the control of the MCK promoter (MCK-Cre). (b): Southern blot analysis of genomic DNA from the liver, SKM (skeletal muscle), WAT (white adipose tissue), and BAT (brown adipose tissue) of control mice and muscleR1KO mice on C57B1/6 background. XbaI-digested mice genomic DNA was hybridized with the probe as shown in (a). (c): Northern blot analysis of Adipor1 mRNA expressed in liver, SKM, WAT, and BAT from control mice and muscle-R1KO mice on C57B1/6 background.

### Mode for Carrying out the Invention

The method of the present invention is a method of screening candidate compounds for adiponectin receptor 1 agonist including a step of judging whether or not test compounds brought into contact with cells cause intracellular influx of extracellular calcium ions and a step of selecting, from the test compounds, compounds causing intracellular influx of extracellular calcium ions as candidate compounds for adiponectin receptor 1 agonist.

The term "compound for adiponectin receptor 1 agonist" as used herein means a compound which, when the compound is bound to adiponectin receptor 1, can cause all or some of various physiological actions caused by binding of adiponectin, which is an internal ligand of adiponectin receptor 1, to adiponectin receptor 1 and can exhibit physiological actions similar to those of adiponectin. The term "candidate agonist compound" means a compound having at least a possibility as an agonist compound.

No particular limitation is imposed on the test compound and any compounds such as oligopeptides, polypeptides, nucleic acids, saccharides, and lipids as well as low-molecular organic compounds can be provided for screening. Since a number of compound libraries having many compounds stored therein have been constructed, it is efficient to provide for screening many compounds registered in such libraries.

The kind of the cells with which the test compounds are brought into contact is not particularly limited and any somatic cell in which AdipoR1 has been expressed can be used. Preferably, cells in which AdipoR1 have been expressed abundantly, for example, skeletal muscle cells can be used. In addition, cells in which AdipoR1 has been expressed by introducing a gene encoding AdipoR1 can be used. In such a case, using, for example, oocytes are also preferred. Cells are however not limited to skeletal muscle cells or oocytes.

When the test compounds are brought into contact with the cells, it is possible to employ a common method such as a method of putting the cells in a medium adjusted to a predetermined calcium ion concentration and adding a solution of each of the test compounds having a predetermined concentration to the resulting medium, but the method is not limited to it.

As a method of detecting influx of extracellular calcium ions into cells, any method that those skilled in the art can employ can be used. In general, influx of extracellular calcium ions into cells can be detected by measuring an increased intracellular calcium ion concentration as a result of influx of calcium ions. Examples of a detecting means include a method of detecting it from fluorescence intensity by using a calcium-ion fluorescent reagent (calcium ion fluorescent probe) which scavenges calcium ions and emits fluorescence and a method of electrophysiologically detecting a current caused by influx of calcium ions into cells, and combined use of these methods. The detection method is however not limited to them.

As the calcium-ion fluorescent reagent, for example, Fluo 4-AM, Fluo 3, Fluo 3-AM, Fura 2, Fura 2-AM, Indo 1, Indo 1-AM, Quin 2, Rhod 2, and Rhod 2-AM are commercially available so that a proper one may be selected as needed, depending on the kind of the cell, kind of the test compound, or the like. For example, Fura 2-AM or the like reagent is preferably used for the measurement of the intracellular calcium ion concentration, because after incorporation into cells from the outside of the cells in the presence of a lipophilic acetoxymethyl group, it is hydrolyzed into Fura 2, coupled to calcium ions, and emits fluorescence of high intensity. It is possible to refer to, for example, Pharmacia, 26, pp.544-546(1990) for a fluorescent reagent for measuring an intracellular calcium ion concentration.

When the calcium ion fluorescent reagent is used, calcium ions entering cells are coupled to a calcium ion fluorescent reagent and thereby fluorescence intensity depending on the calcium ion concentration can be obtained so that the intracellular calcium concentration can be measured with fluorescence intensity as an indicator. For the measurement of fluorescence intensity, any conventional method such as observation under a fluorescent microscope or imaging can be used. It is the common practice to detect an increase in an intracellular calcium concentration from an increase in fluorescence intensity by using a calibration curve.

As a method of electrophysiologically measuring an intracellular calcium concentration, for example, as shown specifically in Examples herein, a current (for example, a current due to chlorine ions activated by the influx of calcium ions) caused by the influx of calcium ions while using cells in which, for example, adiponectin receptor 1 has been expressed can be detected by a common electrophysiological measuring means. The method of electrophysiologically measuring the concentration while using an electrical signal as an indicator is suited for high through-put screening because it can simultaneously detect signals from a large amount of cells and only an inexpensive apparatus is necessary for high-speed detection of electrical signals.

For the measurement of an intracellular calcium ion concentration, it is also preferred to use, as the cells, cells in which adiponectin receptor 1 has been expressed. For this purpose, as described specifically in Examples herein, cells in which adiponectin receptor 1 has been expressed by introducing a gene encoding adiponectin receptor 1 into oocytes or the like cells can be used. However, the kind of cells or method of introducing a gene is not particularly limited.

When from the test compounds, a test compound causing intracellular influx of extracellular calcium ions is found, this test compound can be selected as a candidate compound for adiponectin receptor 1 agonist. This candidate compound is a compound having possibility of inducing all or some of various physiological actions which will be caused by adiponectin, an internal ligand of adiponectin receptor 1, when it is bound to adiponectin receptor 1.

A further preferred mode of the present invention includes a step of bringing test compounds into contact with AdipoR1-deficient cells and AdipoR1 expressing cells to determine whether or not they cause intracellular influx of extracellular calcium ions and a step of selecting, from the test compounds, compounds causing intracellular influx of extracellular calcium ions in the AdipoR1 expressing cells without substantially causing intracellular influx of extracellular calcium ions in the AdipoR1-deficient cells as candidate compounds for adiponectin receptor 1 agonist.

As the AdipoR1-deficient cells, AdipoR1-deficient skeletal muscle cells can be used preferably. It is also possible to use, for example, muscle tissues or myocytes isolated and sampled from an animal (muscle-RIKO animal) produced by muscle-specifically knocking out AdipoR1 by the method described specifically in Examples herein. The method of preparing AdipoR1-deficient cells is however not limited to the above-mentioned particular method. It is needless to say that a desired cell can be prepared by any method usable by those skilled in the art.

According to the above-mentioned preferred mode, test compounds causing intracellular influx of extracellular calcium ions in AdipoR1 expressing cells without substantially causing intracellular influx of extracellular calcium ions in AdipoR1-deficient cells can be selected as a candidate compound for adiponectin receptor 1 agonist. This method is highly precise so that the candidate agonist compound selected using this method is a compound capable of acting as an agonist compound with high possibility.

In a still further preferred mode of the present invention, it is possible to achieve more efficient and highly precise screening by employing, as primary screening, a step of bringing test compounds into contact with cells to determine whether they cause intracellular influx of extracellular calcium ions or not and employing, as secondary screening, a step of bringing the candidate compounds selected by the primary screening into contact with AdipoR1-deficient cells and AdipoR1 expressing cells to determine whether they cause intracellular influx of extracellular calcium ions or not. The above-mentioned two-stage screening is very useful for efficiently selecting agonist compounds for adiponectin receptor 1 from a library including many test compounds.

### Examples

The present invention will hereinafter be described more specifically by Examples. It should however be borne in mind that the scope of the present invention is not limited by the following Examples.

### A. Method

Mice used were from 8 to 10 week old at the time of the experiment. For the measurement of the exercise capacity in muscle-R1KO mice, a treadmill exercise test regimen of 15 m/min for 20 min was used and exercise endurance was assessed by dividing 20 min by the number of times a mouse failed to avoid electrical shocks. Induction of myogenic differentiation using C2C12 cells was carried out according to the method described in the following document (Nature Med. 8, 1288-1295(2002)). By day 5, the cells had differentiated into multinucleated contracting myocytes. C2C12 myocytes were used after myogenic differentiation in all experiments.

The plasmids encoding PGC-1α and PGC-1α-2A mutants were obtained from Dr. B.M. Spiegelman and they are all as described in the following document (Proc. Natl Acad. Sci. USA 104, 12017-12022(2007)). The plasmids encoding PGC-1α-R13 mutants were obtained from Dr. P. Puigserver and they are all as described in the following document (Nature, 434, 113-118(2005)). The results of statistical analysis were indicated by mean ± s.e.m. A difference between two groups was assessed by using unpaired two-tailed t-test. Data including two or more groups were assessed by analysis of variance (ANOVA).
The method will next be described in detail.

### (1) Generation of muscle-R1KO mice

Constructed was a targeting vector for Adipor1 in which a third loxP site had been introduced into the intron 2 of Adipor1 gene and the floxed (loxP-introduced) neomycin-resistant gene (neoR) had been introduced into the intron 5 of the AdipoR1 gene (FIG. 20a). The strategy of culturing, electroporation of J1 embryonic stem (ES) cells (129/Sv), and screening for homologous recombinant clones was as described in J. Biol. Chem. 277, 25863-25866(2002), with slight modifications. Male chimeric mice were mated with C57B1/6 female mice to generate heterozygous (Adipor1^{lox/+}) mice, and F1 progeny from two independently generated male chimeric mice were crossed with C57Bl/6 female mice to obtain F2 mice. Both mice lines showed identical phenotypes in all experiments carried out in this study. Then, the present inventors backcrossed the Adipor1^{lox/+} mice (C57B1/6 and 129/sv background) with C57B1/6 mice more than five times.

Animals transgenically expressing cre recombinase under the control of the muscle creatine kinase promoter (MCK-Cre) were obtained from Dr. C. R. Kahn of the Joslin Diabetes Center (Boston (MA), USA). These animals were backcrossed with C57B1/6 mice more than five times.

The Adipor1^{lox/+} mice were intercrossed with MCK-Cre mice to generate MCK-Cre Adipor1^{lox/+} mice. MCK-Cre Adipor1^{lox/+} mice were crossed with Adipor1^{lox/lox} mice to obtain Adipor1^{lox/+}, MCK-Cre Adipor1^{lox/+}, Adipor1^{lox/lox}, and MCK-Cre Adipor1^{lox/lox} (muscle-R1KO) mice, respectively. The MCK-Cre and Adipor1^{lox/lox} mice were phenotypically indistinguishable; Adipor1^{lox/lox} mice were used as controls. All experiments in this study were conducted on male littermates.

Mice were bred in a cage on a 12-hr dark-light cycle. In all the experiments, a standard feed (CE-2, CLEA Japan) having the following composition: 25.6% (wt/wt) protein, 3.8% fiber, 6.9% ash, 50.5% carbohydrate, 4% fat, and 9.2% water was used (Nature Med., 13, 332-339(2007)).

### (2) Expression levels of AdipoR1 and AdipoR2 in muscle-R1KO mice

Muscle-R1KO mice were generated by crossing animals harboring a floxed AdipoR1 allele with mice that transgenically express cre recombinase under the control of the muscle creatine kinase promoter (MCK-Cre) (Mol. Cell, 2, 559-569(1998)) (FIGS. 26a and 26b). Northern blotting (FIG. 26c) and real-time PCR (data not shown) revealed that in gastrocnemius and soleus muscles of muscle-R1KO mice, AdipoR1 mRNA expression was drastically reduced to about 5% of that see in control mice (FIG. 26c). Other known AdipoR1 expressing tissues such as liver and adipose tissue were also examined, and none were found to have significantly altered AdipoR1 transcript levels (FIG. 26c). AdipoR1 expression was reduced to about 70% in heart but unaltered in non-muscle tissues, indicating that muscle-specific knockout of AdipoR1 was both successful and specific to muscle. Importantly, none of these tissues showed any compensatory upregulation in AdipoR2 levels, and plasma adiponectin levels were not significantly altered.

### (3) Genotyping

Genotyping of the mice was performed by using PCR. Two primers were used for AdipoR1 genotyping: the forward primer was 5'-ACA GGC ACA TAA CAT CTA AGG CA -3' and the reverse primer was 5'-AGC ATG ATA CTG AGT TGG CCA CA-3'. The two primers used for detecting the floxed AdipoR1 gene were 5'-CGG TGT TGA CGA GGC GTC CGA AG -3' and 5'-GGT CTT CGG GAT GTT CTT CCT G -3'. Primers used for detection of Cre were 5'-CGC CGC ATA ACC AGT GAA AC -3', 5'-ATG TCC AAT TTA CTG ACC G-3', 5'-ACA GCG TGA ATT TTG GAG TCA GAA-3', and 5'-GCT TTG CTC AGC CTC GCT AT -3'.

### (4) AMPK phosphorylation in vivo

To study AMPK phosphorylation in vivo, 30 µg of recombinant murine adiponectin per 10 g body weight was intravenously injected into mice through an inferior vena cava catheter (Nature Med. 8, 1288-1295(2002)). This resulted in an increase of plasma adiponectin levels to approximately 30 µg/ml; which showed that the adiponectin dose used in this study was comparable to endogenous adiponectin levels. Mouse full-length adiponectin was generated as described in the following documents (Nature Med. 8, 1288-1295(2002); Nature Med. 7, 941-946(2001); Nature 423, 762-769(2003)).

### (5) Southern blot, northern blot, real-time PCR, and immunoblotting

Northern blot was carried out according to the method described in the following documents (Nature Med. 13, 332-339(2007); Nature 423, 762-769(2003)). Total RNA was prepared from cells or tissues by using Trizol (Invitrogen) according to the manufacturer's instruction. The cDNA probe template of AdipoR1 was prepared by RT-PCR using specific primers. The forward primer used was 5'-GCTGAGGAAGATCAAGCATGCCCGGTG -3' and the reverse primer used was 5'-TTGGAAAAAGTCCGAGAGACCTTCTCTG -3'.

Southern blot was carried out according to the method described in the following documents (Nature Med. 13, 332-339(2007); J. Biol. Chem. 281, 26602-26614(2006)). XbaI-digested mice genomic DNA was hybridized with the probe. The cDNA probe template of AdipoR1 was prepared by RT-PCR using specific primers. The forward primer was 5'-TGTCCACCATCACAGGAAGA'-3 and the reverse primer was 5'-GCTTGCCCTTCTCCTCCA -3'.

Real-time PCR was carried out according to the method described in the following documents (Nature Med. 13, 332-339 2007); Nature 423, 762-769(2003); J. Biol. Chem. 281, 8748-8755(2006)). Total RNA was prepared from cells or tissues by using Trizol (Invitrogen) according to the manufacturer's instructions. Quantification of mRNA was conducted using a real-time PCR method (Nature 423, 762-769(2003)) with slight modifications. The procedures used for immunoblotting are described in the following documents (Nature Med. 7, 941-946(2001); J. Biol. Chem. 281, 26602-26614(2006); J. Biol. Chem. 281, 8748-8755(2006)). The livers or muscles were freeze-clamped in liquid nitrogen in situ (Nature Med. 7, 941-946(2001); J. Biol. Chem. 281, 26602-26614(2006); J. Biol. Chem. 281, 8748-8755(2006)).
Phosphorylation and/or the protein levels of AMPKα, PGC-1α, troponin I, IRS-1, Akt, S6K1, JNK, CaMKI, MEF2, MCAD, SOD2, Catalase, Glut4, HDAC5, and FOXO1 were determined as described in the following documents (EMBO J. 19, 989-996(2000); Nature Med. 7, 941-946(2001); J. Biol. Chem. 281, 26602-26614(2006); J. Biol. Chem. 281, 8748-8755(2006); Nature 458, 1056-1060(2009); Cell Metab. 4, 75-87(2006); Nature 444, 860-867(2006); Steroids 72, 422-428(2007)). Representative data from one of 3-5-independent experiments are shown.

### (6) C2C12 cells

The mouse C2C12 myoblasts were grown in 90% Dulbecco's modified high glucose Eagle's medium containing 10% (v/v) fetal bovine serum. When the cells reached 80% confluent, the myoblasts were induced to differentiate into myocytes by replacing the medium with a low serum differentiation medium (98% Dulbecco's modified high glucose Eagle's medium, 2.5% (v/v) horse serum). The medium was changed daily. The C2C12 cells were transfected by using Pofectamine 2000 (Invitrogen) and following the manufacturer's instructions.

### (7) Mitochondrial content assay

Mitochondrial content assays were carried out as described in the following document (Cell Metab. 4, 75-87(2006)), with slight modifications. Quantification of the mitochondrial content was conducted by using mtDNA and MitoTracker Green probe (Molecular Probes) according to the method as described in the following document (Obes. Res. 13, 574-581(2005)). MitoTracker Green probe preferentially accumulates in mitochondria regardless of the mitochondrial membrane potential and provides an accurate assessment of mitochondrial mass. Cells were washed with PBS and after addition of 100 nM MitoTracker Green FM (Molecular Probes), incubated at 37°C for 30 min. Cells were harvested by using trypsin/EDTA and resuspended in PBS. Fluorescence intensity was detected with excitation and emission wavelengths of 490 and 516 nm, respectively, and the values thus measured were corrected for total protein (mg/ml).

### (8) Histological analyses

The relationship between a change in type I fiber gene expression and actual muscle fiber morphology in the skeletal muscle (soleus) of muscle-R1KO mice was examined by using light microscopy and histochemical analysis. Distinction between type I and type II fibers can be made by myosin ATPase staining at different pH values. In particular, at pH 4.3, type I fibers are well stained while type II fibers are not (Physiol. Rev. 80, 1215-1265(2000)). Samples of the muscle were frozen in liquid nitrogen-cooled isopentane, and transverse serial sections were stained with modified Gomori trichrome (J. Neurol. Sci. 26, 479-488(1975)). Cytochrome c oxidase (COX) (J. Cell Biol. 38, 1-14(1968)) and succinate dehydrogenase (SDH) activities (A Modern Approach, Muscle Biopsy (Saunders, London, 1973)) were enzyme-histochemically analyzed and assessed.

### (9)H₂O₂ Measurement

Plasma H₂O₂ levels were measured by using an Amplex Red hydrogen peroxide assay kit (Invitrogen) (J. Clin. Invest. 118, 789-800(2008)). Mitochondrial H₂O₂ levels were measured by using the Amplex red-horseradish peroxidase method (Molecular probes) (Anal. Biochem. 253, 162-168(1997)).

### (10) Lipid metabolism, lipid peroxidation, and other materials

Skeletal muscle homogenates were extracted and a tissue triglyceride content was determined by the method described in the following documents (Nature Med. 8, 1288-1295(2002); Nature Med. 7, 941-946(2001); J. Biol. Chem. 278, 2461-2468(2003)) with some modifications. To investigate whether oxidative stress increased, lipid peroxidation was measured using a marker of oxidative injury as represented by plasma thiobarbituric acid reactive substance (TBARS) according to the method described in the following document (J. Biol. Chem. 278, 2461-2468(2003)). Tissue samples were homogenized in a buffer solution containing 50 mM Tris-HCl (pH 7.4) and 1.15% KCl, and then centrifuged. The supernatant was used for the assay. The levels of lipid peroxidation in tissue homogenate and plasma were measured in terms of the amount of TBARS by using the LPO test (Wako Pure Chemical Industries). All other materials including chemical substances were obtained from the sources given in the following References (Nature Med. 8, 1288-1295(2002); Nature Med. 7, 941-946(2001); Nature 423, 762-769(2003); J. Biol. Chem. 281, 26602-26614(2006); J. Biol. Chem. 281, 8748-8755(2006); J. Biol. Chem. 279, 30817-30822(2004)).

### (11) Blood sample assays

Plasma glucose levels were determined using a glucose B test (Wako Pure Chemical Industries), while plasma insulin levels were determined using insulin immunoassay (Shibayagi).

### (12) Hyperinsulinemic euglycermic clamp study

Clamp test was carried out as described in the following documents (J. Biol. Chem. 281, 26602-26614(2006); J. Biol. Chem. 281, 8748-8755(2006)), with slight modifications. Two or three days before the test, an infusion catheter was inserted into the right jugular vein under general anesthesia with sodium pentobarbital. The test was performed on mice under conscious and unstressed conditions after 6-hr fasting. A primed continuous infusion of insulin (Humulin R, Lilly) was given (3.0 milliunits/kg/min) and the blood glucose level, monitored every 5 min, was maintained at 120 mg/dl for 120 minutes by administration of glucose (5 g of glucose per 10 ml enriched to 20% with [6,6-²H₂] glucose (Sigma)). Blood was sampled via tail tip after 90, 105, and 120 minutes for determination of the rate of glucose disappearance (Rd). The Rd was calculated according to nonsteady-state equations and endogenous glucose production (EGP) was calculated as a difference between Rd and exogenous glucose infusion rate (J. Biol. Chem. 281, 26602-26614(2006); J. Biol. Chem. 281, 8748-8755(2006)).

### (13) Exercise therapy

The treadmill exercise test was conducted according to the regimen of 15 m/min for 30 min a day for 2 weeks.

### (14) RNA Interference

Two pairs of siRNAs were chemically synthesized, annealed, and transfected into C2C12 myocytes by using Lipofectamine RNAiMAX (Invitrogen) (J. Biol. Chem. 278, 2461-2468(2003)). The sequences of the sense siRNAs are as follows:
mouse AdipoR1: GAGACUGGCAACAUCUGGACATT;
mouse AdipoR2: GCUUAGAGACACCUGUUUGUUTT.
AMPKα1, AMPKα2, CaMKKβ, PGC-1α, SIRT1, and LKB1 siRNA were purchased from Applied Biosystems. Cells transfected with nonfunctional-jumbled siRNA (unrelated) (Applied Biosystems) were used as negative controls. Fourth eight hours after transfection, the cells were lysed.

### (15) NAD⁺/NADH measurements

NAD⁺ and NADH nucleotides were directly measured by the method described in the following document (Nature, 458, 1056-1060(2009)). Whole skeletal muscles or two 10 cm dishes of C2C12 myocytes were homogenized in 200 µl of an acid extraction buffer to measure the NAD⁺ concentration, or 200 µl of alkali extraction to obtain the NADH concentration. Next, homogenates were neutralized and the concentration of nucleotides was measured fluorimetrically after an enzymatic cycling reaction using 5 µl of a sample. Values for both nucleotides were detected within the linear range. NAD⁺/NADH ratios were calculated by comparing the ratios obtained from each animal or from parallel cell dishes in each experiment.

### (16) Calcium imaging in C2C12 myocytes

C212 myocytes cultured on glass base dishes (IWAKI) were treated with 2.5 µM fura-2/AM for 30 min. Fluorescence was measured with an Aquacosmos Calcium Imaging system (Hamamatsu Photonics). Ligands were delivered through the superfusing Ringer's solution (140 mM NaCl, 5.6 mM KCl, 5mM HEPES, 2.0 mM sodium pyruvate, 1.25 mM KH₂PO₄, 2.0 mM CaCl₂, 2.0 mM MgCl₂, 9.4 mM D-glucose (pH 7.4)). For the calcium chelating experiment, EGTA solution (140 mM NaCl, 5.6 mM KCl, 5 mM HEPES, 2.0 mM sodium pyruvate, 1.25 mM KH₂PO₄, 2.0 mM MgCl₂, 5.0 mM EGTA-2Na, 9.4 mM D-glucose (pH 7.4) was used for the bath solution. Fura-2 fluorescence ratios were calibrated to calcium ion signals.

### (17) Gene expression in Xenopus oocytes and electrophysiological recording

Stage V to VII oocytes were treated with 2 mg/ml of collagenase S-1 (Nitta gelatin) in calcium ion-free saline solution (82.5 mM NaCl, 2 mM KCl, 1 mM MgCl₂, and 5 mM HEPES, pH 7.5) for 1 to 2 hr at room temperature. The resulting oocytes were then microinjected with 50 ng of cRNA for mAdipoR1. The cRNA was synthesized from linearized modified pSPUTK. The injected oocytes were incubated for 3 days at 18°C in a bath solution (88 mM NaCl, 1 mM KCl, 0.3 mM Ca(NO₃)₂, 0.4 mM CaCl₂, 0.8 mM MgSO₄, 2.4 mM NaHC0₃, 15 mM HEPES, pH 7.6) supplemented with 10 µg/ml of penicillin and streptomycin.

Whole cell currents were recorded with a two-electrode voltage clamp technique. Intracellular glass electrodes were filled with 3M KCl. Signals were amplified with an OC-725C amplifier (Warner Instruments), low-pass filtered at 50 Hz, and digitized at 1 kHz. A control bath solution contained 115 mM NaCl, 2.5 mM KCl, 1.8 mM CaCl₂, and 10 mM HEPES. It was titrated to pH 7.2 with NaOH. A calcium ion free solution containing 1.8 mM BaCl₂ instead of CaCl₂ was used. The inward current was monitored at a holding potential of -80 mV. Calcium ion activated Cl⁻ current was monitored by applying 500-mscc depolarizing pulses of +100 mV and +50 mV from the holding potential of -80 mV. Adiponectin was supplied to the superfusing bath solution via a silicon tube connected to computer-driven solenoid valves. Data acquisition and analysis were carried out using Digidata 1322A and pCLAMP software.

### (18) Calcium imaging in skeletal muscle

From 8-10 weeks-old mice, soleus muscles were dissected. Preparations were treated with 5 µM fura-2/AM in the presence of 0.1% Chremophore EL (Sigma) for 15 min. Fluorescence images were acquired using an inverted fluorescence microscopy (IX71, Olympus) equipped with a cooled charge-coupled device (CCD) camera (iXon, Andor) and a dry objective lens (10x, NA=0.3, Olympus) through a filter set consisting of a 327-353 nm and 369-391 nm excitation filter, a 409 nm dichroic mirror, and a 515-560 nm fluorescent filter. Ligands were supplied using the superfusing Ringer's solution (150 mM NaCl, 4 mM KCl, 2.0 mM CaCl₂, 1.0 mM MgCl₂, 30 mM D-glucose, 5 mM HEPES (pH 7.4)).

### (19) Insulin resistance index

Oral glucose tolerance test (OGTT) and insulin tolerance test (ITT) were conducted by the method described in the following documents (Nature Med. 8, 1288-1295(2002); Nature Med. 7, 941-946(2001)), with some modifications. The areas of the glucose and insulin curves were calculated, respectively, by multiplying the cumulative mean height of the glucose values (1 mg/ml=1 cm) and insulin values (1 ng/ml=1 cm) by time (60 min=1 cm) (Nature Med. 13, 332-339(2007); Nature Med. 7, 941-946(2001)). The insulin resistance index was calculated from the product of the areas of glucose and insulin x 10⁻² in the glucose tolerance test. The results were expressed as the percentage of the value of control littermates.

### (20) Citrate synthase activity

Citrate synthase activity was determined from the homogeneates by using a spectrophotometer according to the method described in the following documents (Cell Metab. 4, 75-87(2006); Eur. J. Biochem. 10, 198-206(1969); Obes Res 13, 574-581(2005)). The citrate synthase activity was measured at 37°C in 0.1M Tris-HCl (pH 8.3) assay buffer containing 0.12 mM 5,5'-dithio-bis(2-nitrobenzoic acid) and 0.6 mM oxaloacetate. After recording of absorbance at 412 nm in the first two minutes, the reaction was started with the addition of 3 mM acetyl-CoA and a change in absorbance was measured every 10 sec for 7 min.

### (21) Oxygen consumption

Oxygen consumption of animals under the fasting condition was measured using an O₂/CO₂ metabolism measurement system every 3 min for 24 hr (Model MK-5000; Muromachi Kikai, Tokyo, Japan). Each mouse was placed in a hermetically sealed chamber (560 ml volume) with an air flow of 0.5 l/min. The oxygen consumed was converted to milliliters/minute by multiplying it by the flow rate.

### (22) Increased calcium influx experiment

Hydrogels were prepared by crosslinking aqueous gelatin solutions with glutaraldehyde according to the method described in the following document (J. Control Release 64, 133-142(2000)). For incorporating Bay-K 8644 into the resulting gelatin hydrogels, 20 µl of Bay-K 8644 (0.25 µM) was added dropwise onto 20 mg of freeze-dried hydrogel and left it overnight at 4°C. Under anesthesia, the gelatin hydrogel having Bay-K 8644 incorporated therein was implanted under the skin. Then, the skin was carefully sutured with No. 4-0 nylon monofilament.

### (23) Patch clamp test

Whole-cell currents were amplified with a patch-clamp amplifier (CEZ-2400; Nihon Kohden) and were digitized with a PowerLab digitizer (AD Instruments). Ringer's solution was used for the extracellular solution. The electrode solution contained 140 mM KCl, 10 mM HEPES, 5 mM EGTA-2K, and 10 mM D-glucose (pH 7.4). The data were low-pass filtered at 2 kHz and were sampled at 20 kHz. Ligands dissolved in Ringer's solution were applied at least twice with a self-made pressure-ejection system and via a two-barreled glass capillary.

### B. Results

### (1) Decreased PGC-1α and mitochondria in muscle-R1KO mice

Muscle-R1KO mice showed decreased phosphorylation of AMPK (FIG. 1a). Moreover, administration of adiponectin increased the phosphorylation of AMPK in the muscle of control littermates, but not that in the muscle of muscle-R1K0 mice (FIG. 7a). On the other hand, adiponectin increased the phosphorylation of AMPK in the liver of both genotypes (FIG. 7b). In the muscle-R1K0 mice, the molecules involved in mitochondrial biogenesis such as PGC1-α (Cell 98, 115-124(1999)) were also decreased at both messenger RNA (Ppargcla) (FIG. 1b) and protein levels (FIG. 1c). Adiponectin increased the expression levels of Ppargcla in the muscle of control littermates but not those in the muscle of muscle-R1KO mice (FIG. 7c).

In the muscle-R1KO mice, a decrease in the molecules involved in mitochondrial biogenesis such as estrogen-related receptor α (Esrra) (Proc. Natl Acad. Sci. USA 101, 6570-6575(2004)), the molecules involved in transcription such as nuclear respiratory factor 1 (Nrf1), and the molecules involved in mitochondrial DNA replication/translation such as mitochondrial transcription factor A (Tfam) was observed (FIG. 1b). Moreover, the expression levels of a number of oxidative phosphorylation and other mitochondrial genes were significantly reduced in muscle-R1KO mice. They contained cytochrome c (Cycs) and cytochrome c oxidase subunit II (mt-Co2) (FIG. 1b). In addition, the mitochondrial DNA content of the muscle-R1K0 mice was decreased (FIG. 1d).

The mitochondrial function was assessed by measuring the enzymatic activities of Cox (FIG. 8a) and succinate dehydrogenase (SDH) (FIG. 8b). Staining of soleus muscle sections has revealed a smaller number of Cox- and SDH-positive muscle fibers and a decreased intensity of Cox and SDH staining in muscle-R1KO mice (FIGS. 8a and 8b).

### (2) Decreased type I fibers and exercise capacity in muscle-R1KO

In muscle-R1KO mice, the molecules involved in type I fiber (Physiol. Rev. 80, 1215-1265(2000)) differentiation myocyte enhancer factors 2C (Mef2c) (EMBO J. 19, 1963-1973(2000)) (FIG. 1b) were decreased and also type I fiber marker troponin I (slow) was decreased (FIG. 1e). In contrast, the expression levels of MHCIIa, MHCIIx, and MHCIIb in muscle-R1K0 mice were almost similar to those seen in control mice (FIGS. 8c to 8e), indicating a reduction in oxidative, high endurance fibers in muscle-R1K0 mice. These findings were consistent with the histological analysis (FIGS. 1f and 1g). In the soleus muscle of muscle-R1KO mice, type I fibers were decreased by 20% (FIG. 1g).

To study the impact of AdipoR1 ablation on skeletal muscle function in intact animals, muscle endurance test by tread mill running for assessing the involuntary physical exercise was conducted in control and muscle-R1KO mice. The muscle endurance of muscle-R1KO mice was significantly lower than that of the control mice (FIG. 1h). Next, as a result of examination of the expression of metabolic genes, it has been found that the molecules involved in fatty acid oxidation, such as medium-chain acyl-CoA dehydrogenase (Acadm) was significantly decreased in muscle-R1KO mice (FIG. 1b), which was associated with decreased β oxidation (FIG. 1i) and increased triglyceride content (FIG. 1j) in skeletal muscle.

In muscle-R1KO mice, the expression levels of both mitochondrial and cytoplasmic oxidative stress-detoxifying genes such as manganese superoxide dismutase (Sod2) (FIG. 1b) and catalase (Cat) (FIG. 1b) were decreased and oxidative stress such as thiobarbituric acid reactive substance (TBARS) was increased in muscle (FIG. 1k). The expression of insulin-sensitive glucose transporter 4 (Slc2a4) was decreased in muscle-R1K0 mice (FIG. 1b).

### (3) Insulin resistance in muscle-R1KO mice

The glucose levels and plasma insulin levels after administration of glucose were significantly higher in muscle-R1KO mice than in control mice (FIGS. 2a and 2b). The capacities of muscle-R1KO mice of regulating plasma glucose levels were significantly decreased after ingestion of insulin (FIG. 2c). As a result of hyperinsulinemic euglycermic clamp experiment, disruption of AdipoR1 in muscle did not significantly alter endogenous glucose production, but significantly decreased a glucose disposal rate (Rd) and a glucose infusion rate (GIR), indicating decreased insulin sensitivity in muscle (FIGS. 2d to 2f).

In agreement with the data obtained from the hyperinsulinemic euglycermic clamps, decreased tyrosine phosphorylation of IRS-1 (FIG. 2g) and decreased phosphorylation of Ser 473 in Akt (FIG. 2h) were found in the skeletal muscle of muscle-R1KO mice, which was presumed to be associated with increased phosphorylation of S6K1 (FIG. 2i) and JNK (FIG. 2j). It has been reported that Ser 302 in mice IRS-1 is phosphorylated by JNK (Nature 444, 860-867(2fly)) and phosphorylation of Ser 636/639 is mediated by mTOR and S6K1 pathways (Nature 431, 200-205(2004)), both of which would result in inhibition of insulin signaling. The amounts of phosphorylation of Ser 302 and Ser 636/639 in IRS-1 were increased in the skeletal muscle of muscle-R1KO mice (FIGS. 2k and 21).

### (4) CaMKKβ is required for adiponectin/AdipoR1-induced PGC-1α

Next, the molecular mechanisms by which muscle-R1KO mice showed the above-mentioned phenotypes were studied. Incubation of C2C12 myocytes added with 30 µg/ml adiponectin increased a mitochondrial DNA content (FIG. 3a). AMPK activity depends on phosphorylation of AMPKα (Thr 172) in its activation loop by AMPK kinase (AMPKK) (Cell Metab. 1, 15-25(2005); J. Biol. Chem. 271, 27879-27887(1996)). LKB1 and CaMKKβ are known to be two main AMPKKs present in various tissues and cells (Cell Metab. 1, 15-25(2005); Cell Metab. 2, 9-19(2005); Cell Metab. 2, 21-33(2005)). Suppression of AdipoR1 or CaMKKβ or suppression of the expression of both AMPKα1 and AMPKα2 or PGC-1α by respective small interfering RNAs (siRNAs) (FIGS. 9a to 9e) specific to them markedly reduced an increase in mitochondrial DNA content induced by adiponectin (FIG. 3a). In contrast, even suppression of expression of AdipoR2 by specific siRNA (FIG. 9f) failed to significantly reduce the mitochondrial biogenesis induced by adiponectin (FIG. 3a).

Suppression of the expression of AdipoR1 or CaMKKβ by respective specific siRNAs (FIGS. 9a and 9b) greatly reduced an increase in PGC-1α expression induced by adiponectin (FIG. 3b). Interestingly, the suppression of AMPKα1 and AMPKα2 expression (FIGS. 9c and 9d) by respective specific siRNAs failed to significantly reduce the PGC-1α expression induced by adiponectin (FIG. 3b), suggesting that the PGC-1α expression was AdipoR1- and CaMKKβ- dependent, but it was induced by adiponectin via an AMPK-independent pathway.

It has been reported that PGC-1α is activated after phosphorylation by AMPK (Proc. Natl Acad. Sci. USA 104, 12017-12022(2007)) or activated by deacetylation through SIRT1 activation (Nature 434, 113-118(2005)). Therefore, the possibility of AMPK, which had been activated by adiponectin and AdipoR1, regulating PGC-1α activities was studied. Treatment of C2C12 myocytes with adiponectin decreased PGC-1α acetylation after treatment for 2 hr (FIG. 3c). Suppression of AdipoR1, both AMPKα1 and AMPKα2, or SIRT1 expression (FIGS. 9a, 9c, 9d, 9g) by respective siRNAs specific to them largely reduced the decrease in PGC-1α acetylation induced by adiponectin (FIG. 3c). The PGC-1α-2A mutant lacking two AMPK phosphorylation sites (Proc. Natl Acad. Sci. USA 104, 12017-12022(2007)) showed a marked decrease in PGC-1α deacetylation and mitochondrial biogenesis induced by adiponectin (FIGS. 3d and 3e).

In C2C12 myocytes expressing PGC-1α-R13 in which 13 of the potential acetylation sites of lysine was mutated into arginine (Nature 434, 113-118(2005)), adiponectin failed to further induce mitochondrial biogenesis (FIG. 3f). Since the capacity for undergoing acetylation is impaired in the PGC-1α-R13 mutant, these data are consistent with the dependence of adiponectin on SIRT11-mediated deacetylation of PGC-1α in activating PGC-1α.

SIRT1 deacetylase activity has been reported to depend on the NAD⁺ levels (Nature 434, 113-118(2005); Nature 444, 868-874(2006)). Adiponectin increased a NAD⁺/NADH ratio in C2C12 myocytes (FIG. 3g). In the muscle-R1KO mice, increased PGC-1α acetylation (FIG. 3h) and decreased NAD⁺/NADH ratio in vivo upon stimulation with adiponectin (FIG. 3i) were found. These data have suggested that the total activity as assessed by multiplying expression and deacetylation of PGC-1 is markedly reduced in muscle-R1KO mice (FIG. 10).

### (5) Adiponectin induces calcium ion influx via AdipoR1.

Interestingly, treatment of C2C12 myocytes with adiponectin increased an intracellular calcium ion concentration as measured by fura-2-based fluorescent imaging (FIG. 4a). C2C12 myocytes showed dose-dependent responses to adiponectin. Removal of extracellular free calcium ions by EGTA almost completely abolished the adiponectin-induced calcium ion response (FIG. 4a), whereas EGTA had no effect on the ATP-induced intracellular calcium ion release. Suppression of AdipoR1 expression by using specific siRNA (FIG. 4b) largely reduced the calcium response of C2C12 myocytes to adiponectin (FIGS. 4c and 4d). These results suggest that the influx of extracellular calcium ions after treatment of C2C12 myocytes with adiponectin is mediated by AdipoR1.

In order to study further the importance of AdipoR1 in adiponectin-induced calcium ion responses in the gain of function study system, AdipoR1 was expressed in *Xenopus* oocytes (FIG. 4e). An increase in intracellular calcium levels caused by stimulation with adiponectin was detected by monitoring calcium-ion activated Cl⁻ current in oocytes injected with AdipoR1 complementary RNA (FIGS. 4f and 4g). The responses were not observed in control oocytes (FIG. 4g). As a result of a study of the effect of removal of extracellular free calcium ions by EGTA on the calcium-ion activated Cl⁻ current responses to adiponectin in *Xenopus* oocytes expressing AdipoR1, it was found that removal of extracellular free calcium ions by EGTA almost completely abolished the calcium-ion activated Cl⁻ current responses to adiponectin in *Xenopus* oocytes expressing AdipoR1 (FIG. 4g), indicating that these responses were dependent on extracellular calcium ions.

### (6) Adiponectin-induced calcium ion influx is important for action expression.

Incubation of C2C12 myocytes after addition of adiponectin thereto increased the phosphorylation of the AMPK a-subunits at Thr 172 and EGTA partially suppressed an increase in AMPK phosphorylation induced by adiponectin (FIG. 5a). EGTA almost completely abolished ionomycin-dependent phosphorylation of AMPK but EGTA had no effect on phosphorylation of AMPK induced by 5-aminoimidazole-4-carboxamide-1-β-D-libocide (AICAR) in C2C12 myocytes (FIG. 5a).

Suppression of CaMKKβ or LKB1 expression by respective siRNAs specific to them (FIGS. 9b and 9h) significantly reduced an increase in phosphorylation of AMPK induced by adiponectin (FIG. 5b). Although AraA, an AMPK inhibitor, only tended to decrease PGC-1α expression induced by adiponectin, calcium ion removal by EGTA or inhibition of CAMKKβ with STO-609 (J. Biol. Chem. 277, 15813-15818(2002)), a selective inhibitor, effectively and almost completely abolished an increase in PGC-1α expression stimulated with adiponectin in C2C12 myocytes (FIG. 5c) as removal of extracellular calcium ions effectively abolishes calcium ion signals induced by adiponectin (FIG. 4a).

Whether adiponectin-induced calcium ion influx into skeletal muscle decreased in mucle-R1KO mice or not was studied by in vivo imaging (Am. J. Physiol. Heart Circ. Physiol. 275, H1652-H1662(1998); J. Biol. Chem. 281, 1547-1554(2006)). Treatment of soleus muscle with adiponectin increased the intracellular calcium ion concentration in control mice as measured by fura-2-base fluorescent imaging. On the other hand, calcium responses of soleus muscle to adiponectin almost completely disappeared in muscle-R1KO mice (FIGS. 5d to 5f). These results are consistent with the observation that adiponectin significantly increased the phosphorylation of CaMKI37 and 38, intracellular substrates of CaMKKβ, via AdipoR1 in skeletal muscle in vivo (FIG. 11).
Whether or not simultaneous activation of calcium ion signaling pathway and AMPK/SIRT1 pathway by exercise could rescue phenotype in muscle-R1KO mice independent of AdipoR1 was studied. Two weeks' exercise significantly ameliorated insulin resistance and increased mitochondrial content and function such as citrate synthase activities in the muscle of muscle-R1KO mice (FIGS. 6a to 6d).

The results will next be described in detail.

### (7) Muscle-R1KO deteriorates adiponectin action in skeletal muscle.

Muscle-R1KO exhibited decreased phosphorylation of AMPK (FIG. 1a) and its intracellular substrate, that is, acetyl CoA carboxylase (ACC) (FIG. 14a). Muscle-R1KO showed decreased mitochondria-specific Gomori staining (FIG. 15a).

### (8) Decreased type I fibers in muscle-R1KO

Muscle-R1KO exhibited a decrease in molecules involved in type I fiber differentiation myocyte enhancer factor 2C (Mef2c) (EMBO J. 19, 1963-1973(2000)) (FIGS. 1b and 16a) and also type I fiber marker myosin heavy chain (MHC) isoform I (FIG. 15b), troponin I (slow) (Tnni1) (FIGS. 1e and 15c) and myoglobin (Mb) (Physiol. Rev. 80, 1215-1265(2000)) (FIG. 15d). These findings were consistent with the histological analysis (FIGS. 1f, 1g, and 15e). In soleus muscle of muscle-R1KO mice, type I fibers were decreased by 20% (FIGS. 1g and 15e).

### (9) Protein expression levels of MEF2, MCAD, SOD2, catalase, and Glut4 in the muscle of muscle-R1KO mice

The protein expression levels of MEF2, MCAD, SOD2, catalase, and Glut4 in the muscle of muscle-R1KO mice were significantly decreased compared with those seen in control mice (FIG. 16). These results were consistent with the results that muscle-R1KO mice exhibited a decrease in type I fibers, an increase in tissue triglyceride content, and an increase in oxidative stress in their muscle.

### (10) Increased amount of mitochondria in C2C12 myocytes treated with adiponectin

Incubation of C2C12 myocytes with 30 µg/ml adiponectin increased a mitochondrial DNA content and amount of mitochondria as assessed by using MitoTracker green (FIGS. 17a to 17c). AraA, an AMPK inhibitor, alone almost completely abolished the effect of adiponectin on the increase in mitochondrial biogenesis (FIGS. 17a and 17c). STO-609 (J. Biol. Chem. 277, 15813-15818(2002)), a CAMKK inhibitor, almost completely abolished the effect of adiponectin on the increase in mitochondrial biogenesis.

### (11) NAD⁺ and NADH contents in C2C12 myocytes and skeletal muscle

Adiponectin increased the NAD⁺/NADH ratio and NAD⁺ and NADH contents in C2C12 myocytes (FIGS. 3g, 18a, and 18b). Muscle-R1KO also exhibited a decrease in NAD⁺/NADH ratio and a decrease in NAD⁺ and NADH contents in vivo (FIGS. 3i, 18c and 18d). These results suggest that total activity as assessed by multiplying expression and deacetylation of PGC-1α is markedly reduced in muscle-R1KO mice (FIG. 10).

### (12) PPARα levels and activity in skeletal muscle of muscle-R1KO mice

Muscle-specific disruption of AdipoR1 had no significant effect on PPARα (Ppara) levels (FIG. 19a), however, it resulted in significantly decreased expression levels of PPARα target genes such as ACO (Acox1) in the muscle (FIG. 19b), suggesting deterioration in PGC-1α activity. These results were consistent with the results that muscle-R1KO mice exhibit decreased PGC-1α activity as PPAR co-activation in muscles.

### (13) Oxygen (O₂) consumption and respiratory quotient (RQ) of muscle-R1KO mice

Muscle-specific disruption of AdipoR1 significantly decreased O₂ consumption (FIG. 19c), whereas it had no significant effect on RQ (FIG. 19d). These results suggest that muscle-specific disruption of AdipoR1 resulted in decrease in both glucose and fatty acid oxidation.

### (14) The mRNA levels of Glut 4 in gastrocnemius and soleus muscles

The expression levels of Glut4 (Slc2a4) were significantly decreased in muscles rich in type I fibers such as soleus muscle and also muscles rich in type II fibers such as gastrocnemius of muscle-R1KO as compared with those in control mice (FIGS. 19e and 19f). These results are consistent with the notion that the observed reduction of Glut4 in the muscle of muscle-R1KO mice is not simply secondary due to alteration of the fiber-type composition.

### (15) The effect of increased calcium influx in muscle of muscle-R1KO mice

An increase in CaMKKβ activity caused by the addition of 0.25 µM Bay-K 8644, a calcium ion channel opener (Nature 303, 535-537(1983); Am. J. Physiol. 258, R462-468(1990)), (FIG. 20a) significantly enhanced, in the muscle of muscle-R1KO mice, insulin-stimulated phosphorylation of Akt (FIG. 20b), mitochondrial content (FIG. 20c), and function such as citrate synthase activities (FIG. 20d). These results are consistent with the conclusion that adiponectin-induced influx of extracellular calcium ions via AdipoR1 is important for an adiponectin induced increase of mitochondrial biogenesis in muscle.

### (16) The effect of exercise, AICAR, resveratrol, and antioxidant MnTBAP on muscle-R1KO mice

Administration of AICAR (Am. J. Physiol. 273, E1107-1112(1997)) significantly increased the phosphorylation of AMPK in the muscle of muscle-R1KO mice (FIG. 20e). Activation of AMPK via AICAR (Diabetologia 45, 56-65(2002); Cell 134, 405-415 (2008)) and activation of SIRT1 via resveratrol (Cell 127, 1109-1122(2006)) significantly ameliorated insulin resistance and increased a mitochondrial content and function such as citrate synthase activities in the muscle of muscle-R1KO mice (FIGS. 20f to 20i and 21a to 21d). These results are consistent with the conclusion that adiponectin-stimulated AMPK and SIRT1 activations via AdipoR1 are important for adiponectin-derived increases in insulin sensitivity and mitochondrial biogenesis in muscle.

Although MnTBAP (Nature 440, 944-948 (2006)) indeed significantly decreased ROS such as H₂O₂ (FIG. 21e), MnTBAP alone tended to ameliorate insulin resistance and failed to significantly increase the mitochondrial content and function such as citrate synthase activities in the muscle of muscle-R1KO mice (FIGS. 21e to 21i). These results suggest the possibility that antioxidative stress-independent pathways such as inhibition of S6K1 by AMPK and inhibition of tissue triglyceride accumulation, together with antioxidative stress dependent pathways, contribute largely to the insulin sensitivity enhancing effects of adiponectin/AdipoRl in muscle. Antioxidative stress-independent pathways such as increased NRF-1 (FIG. 1b) and mtTFA (FIG. 1b) by AMPK/SIRT1 are presumed to largely contribute to mitochondrial biogenesis by adiponectin/AdipoRl in muscle.

AICAR significantly but partially rescued the AdipoR1 loss in muscle (FIG. 20f to 20i), but exercise significantly and almost completely rescued it (FIGS. 6a to 6d). These results may be explained by several mechanisms. First, exercise could activate AMPK more efficiently than AICAR. In skeletal muscle, AMPK can be activated via two different pathways, that is, LKB1 dependent phosphorylation and CaMKKβ dependent phosphorylation. AICAR has been shown to activate LKB1-dependent phosphorylation and activation of AMPK as AMP mimetics inducing a structural change of AMPK that allows efficient phosphorylation by ordinarily structurally activated LKB1, it does not stimulate CaMKKβ-dependent phosphorylation and activation since AICAR does not elevate an intracellular calcium ion concentration. On the other hand, exercise has been shown to elevate both intracellular AMP and calcium ion concentration so that it can stimulate both LKB1-dependent and CaMKKβ-dependent phosphorylation and activation of AMPK. Second, exercise, but not AICAR, has been shown to stimulate enhancement of intracellular calcium ion concentration-dependent yet AMPK-independent pathways, such as activation of the members of CaMK and subsequent increase in the expression of exercise-regulated muscle genes (particularly PGC-1α) (Nature 454, 463-469(2008)), which is presumed to contribute to rescue the AdipoR1 loss in muscle.

### (17) Mitochondrial capacity and insulin signaling

Suppression of Adipor1 by specific siRNA (FIG. 9a) markedly reduced the increase in mitochondrial content by adiponectin (FIG. 3a) and at the same time, markedly reduced the increase in insulin-stimulated phosphorylation of Ser 473 in Akt (FIG. 22a). These data are consistent with the notion that activation of mitochondrial capacity especially by adiponectin is associated with increased insulin signaling in myocytes.

### (18) FOXO1 phosphorylation in skeletal muscle of muscle-R1KO mice

Insulin-stimulated phosphorylation of Thr 24 and Ser 253 in Foxo (EMBO J. 19, 989-996(2000)) was markedly decreased in the skeletal muscle of muscle-R1KO mice as compared with that in control mice (FIG. 22b). These results are consistent with the notion that many of the effects of AdipoR1 knockout in muscle such as decreased type I myofibers, poor glycemic control, and low capacity for physical exercise are similar to the effects of Foxo activation (J. Biol. Chem. 279, 41114-41123 (2004)).

### (19) The amounts of PGC-1α acetylation in adiponectin-stimulated C2C12 myocytes treated with deacetylase SIRT1 knockdown

The amounts of PGC-1α acetylation in adiponectin-stimulated C2C12 myocytes treated with deacetylase SIRT1 knockdown were almost the same as those in C2C12 myocytes without adiponectin stimulation. These results show the possibility that under the conditions of the present inventors such as 6-hr fasting, deacetylase SIRT1 may not be sufficiently activated in C2C12 myocytes without adiponectin stimulation (FIG. 22c).

### (20) Inward current responses to adiponectin

Inward current responses to adiponectin were observed by whole-cell patch-clamp recordings of C2C12 myocytes at a holding potential of -60 mV (FIG. 23a). In addition, suppression of AdipoR1 expression by specific siRNA (FIG. 4b) markedly reduced the whole-cell current responses to adiponectin of C2C12 myocytes (FIGS. 23a and 23b). These results suggest that current generation upon the adiponectin treatment of C2C12 myocytes is mediated by AdipoR1.

Stimulation of oocytes expressing AdipoR1 with adiponectin induced inward current responses (FIGS. 23c and 23d). As a result of studying the effect of the removal of extracellular free calcium ions by EGTA on the inward current responses induced in *Xenopus* oocytes expressing AdipoR1 by adiponectin stimulation, it has been found that removal of extracellular free calcium ions by EGTA almost completely abolishes the adiponectin-induced inward current responses in *Xenopus* oocytes expressing AdipoR1 (FIG. 23d). The above-mentioned results show that these responses were dependent on extracellular calcium ions.

### (21) Phosphorylation of AMPK stimulated with adiponectin in the presence of BAPTA-AM or U73122

Bis-(o-aminophenoxy)ethane-N,N,N9,N9-tetra-acetic acid, tetraacetoxymethyl ester (BAPTA-AM) (intracellular calcium chelator) partially suppressed adiponectin-induced increased AMPK phosphorylation to almost the same extent as EGTA (FIG. 24a), whereas U73122 (PLC inhibitor) had little effect on adiponectin-induced phosphorylation of AMPK in C2C12 myocytes (FIG. 24b).

### (22) Sequential phosphorylation of CaMKI, AMPK, and HDAC5 and their extracellular calcium ion influx dependency

Treatment of C2C12 myocytes with adiponectin caused a rapid, robust, and transient increase in CaMKI phosphorylation (J. Biol. Chem. 273, 31880-31889(1998); Trends Biochem. Sci. 24, 232-236(1999)) (FIGS. 25a and 25b upper panel and FIG. 25c). Removal of extracellular calcium ions by EGTA almost completely abolished the increased CaMKI phosphorylation stimulated with adiponectin (FIGS. 25a and 25b upper panel, FIG. 25c). These data indicate that the adiponectin-induced CaMKI phosphorylation presumably through CaMKKβ activation almost completely depend on calcium ion entry. Subsequent to the phosphorylation of CaMKI, adiponectin increased AMPK phosphorylation further in a sequential and partial manner depending on extracellular calcium ion influx (FIGS. 25a and 25b center panel, FIG. 25c).

Next, whether adiponectin stimulates phosphorylation of histone deacetylase (HDAC) 5 subsequent to the activation of CaMKI (Nature 408, 106-111(2000)) and AMPK (Diabetes 57, 860-867(2008)) was examined. CaMK and/or AMPK signaling prevent formation of MEF2-HDAC complexes and induce nuclear export of HDAC5 by phosphorylation of this transcriptional repressor (Nature 408, 106-111(2000); Diabetes 57, 860-867(2008)). These signaling pathways may be mechanisms by which CaMK and/or AMPK increases PGC-1α expression via MEF2 in myocytes. The treatment of C2C12 myocytes with adiponectin increased HDAC5 phosphorylation in a sequential and partial manner depending on extracellular calcium ion influx (FIGS. 25a and 25b lower panel, FIG. 25c). Incubation for from 20 to 40 minutes in a calcium ion-free and EGTA-added medium without adding adiponectin (matched controls) had no significant effects on the phosphorylation amounts of CaMKI, AMPK, and HDAC5 (FIGS. 25a to 25c).

### (23) Comparison between insect olfactory receptors and AdipoR1

It has been reported that insect olfactory receptors lack homology to GPCR, possess an opposite seven-transmembrane topology with the amino terminus located intracellularly and induce extracellular calcium ion influx (Nature 452, 1002-1006(2008)). There is therefore the possibility that signaling by AdipoR1 is similar to signaling of insect olfactory receptors. The above-mentioned results suggest the development of new antidiabetic and anti-atherogenic drugs with AdipoR1 as a molecular target.

### (24) Differences between PGC-1α and adiponectin/AdipoR1 in the regulation of insulin sensitivity

PGC-1α is a key regulator of oxidative metabolism in skeletal muscle. Reduced PGC-1α and OXPHOS levels have been associated with insulin resistance and type 2 diabetes in humans (Nature Genet. 34, 267-273(2003)) and mice (FIGS. 14a to 14c). However, muscle-specific PGC-1α-knockout mice did not exhibit insulin resistance (J. Clin. Invest. 117, 3463-3474(2007)). Also PGC-1α-independent pathways such as inhibition of S6K1 by AMPK are presumed to contribute to the insulin sensitizing effects of adiponectin/AdipoR1 in muscle.

### (25) Conclusion

As described above, muscle-R1KO mice exhibited a decreased mitochondrial content and enzyme. PGC-1α is a key regulator of a mitochondrial content and function. It has been reported that PGC-1α expression is modulated in several physiological conditions. For example, in skeletal muscle, it is modulated by increased calcium ion signaling via molecules such as CaMK and CREB in partial response to exercise (Nature 454, 463-469(2008)). It has also been reported that PGC-1α activities are modulated by several kinds of PGC-1α modifications, such as phosphorylation via AMPK (Proc. Natl Acad. Sci. USA 104, 12017-12022(2007)) and deacetylation via AMPK and SIRT1 (Nature 434, 113-118(2005)). AMPK and SIRT1 could also be activated by exercise. It has been proved that muscle-R1KO mice exhibited decreased PGC-1α expression as well as decreased PGC-1α acetylation. Consistent with it, it has also been proved that adiponectin induces calcium ion influx via AdipoR1 and thereby activates CaMKKβ and causes increased PGC-1α expression and at the same time, adiponectin/AdipoRl activates AMPK and SIRT1, which induces deacetylation of PGC-1α. These data suggest that adiponectin and AdipoR1 stimulate increases in both the expression and activation of PGC-1α in a similar manner to exercise (FIG. 6e).

Although the decreasing degree of PGC-1α expression was approximately 40% in muscle-R1KO mice, the decreasing degrees of mitochondrial biogenesis and exercise endurance were comparable to those observed in muscle-specific PGC-1α-knockout mice (J. Biol. Chem. 282, 30014-30021(2007)), which may be explained by the finding that adiponectin and AdipoR1 increase not only PGC-1α expression but also PGC-1α activities (FIG. 6e).

Decreases in the expression of AdipoR1 and PGC-1α and mitochondrial DNA content were also observed in type 2 diabetic patients (Nature Genet. 34, 267-273(2003)) and individuals at increased risk of developing diabetes due to their family history (Proc. Natl Acad. Sci. USA 100, 8466-8471(2003)) as well as in obese diabetic db/db mice (FIGS. 12a to 12c). These data suggest that decreased adiponectin/AdipoRl in pathophysiological conditions such as obesity may be a cause of PGC-1α dysregulation and mitochondrial dysfunction.

In skeletal muscle, AdipoR1 is involved in regulation of insulin sensitivity via multiple mechanisms (FIG. 13). First mechanism is the activation of S6K1, which has been reported to be able to provide insulin resistance via increased Ser 636/639 phosphorylation of IRS-1 (Nature 431, 200-205(2004)). S6K1 is crucially inhibited by AMPK (J. Biol. Chem. 282, 7991-7996(2007)). The AMPK activation was reduced in the skeletal muscle of muscle-R1KO mice, but activation of S6K1 and Ser 636/639 phosphorylation of IRS-1 were indeed increased. Second mechanism is oxidative stress increase, which has been linked as a cause of insulin resistance (Nature 440, 944-948(2006)) via increased Ser 302 phosphorylation of IRS-1 through JNK activation (Nature 444, 860-867(2006)). Some oxidative stress-detoxifying genes were crucially regulated by AMPK and PGC-1α (Cell 127, 397-408(2006)) and the expression levels of these genes such as Sod2 and cat were reduced, which has been associated with increased TBARS in the skeletal muscle of muscle-R1KO mice. Third mechanism is an increased triglyceride content, which is associated with insulin resistance via increased Ser 302 phosphorylation of IRS-1 through JNK activation (Nature 444, 860-867(2006)). Molecules involved in fatty acid oxidation are crucially regulated by AMPK and PGC-1α and the expression levels of these genes such as Mcad were reduced, which has been associated with an increased triglyceride content in the skeletal muscle of muscle-R1KO mice. JNK activation and Ser 302 phosphorylation in IRS-1 were indeed increased, consistent with an increase in TBARS and triglyceride content.

It has been reported that exercise has beneficial effects on longevity and life-style related diseases, and at the same time on activation of calcium ions, AMPK, SIRT1, and PGC-1α pathways (Nature 454, 463-469(2008)). It has been demonstrated that adiponectin and AdipoR1 regulate PGC-1α and mitochondria via calcium ions and AMPK/SIRT1. Therefore, the agonism of AdipoR1 as well as strategies of increasing AdipoR1 in muscle could be pseudo-exercise effect.

In conclusion, AdipoR1 plays a critical role in the physiological and pathophysiological significance of adiponectin in muscle and are involved in the regulation of calcium ion signaling, PGC-1α expression and activation, mitochondrial function and oxidative stress, glucose and lipid metabolism, and exercise endurance. The above-mentioned results suggest that the agonism of AdipoR1 as well as strategies of increasing AdipoR1 in muscle may provide a new treatment method for mitochondrial dysfunction, insulin resistance, and type 2 diabetes linked to obesity.

## Claims

1. A method of screening for candidate compounds for adiponectin receptor 1 agonist, comprising bringing test compounds into contact with cells to determine whether or not the test compounds cause intracellular influx of extracellular calcium ions and selecting, from the test compounds, compounds causing the intracellular influx of extracellular calcium ions as the candidate compounds for adiponectin receptor 1 agonist.

2. The method according to Claim 1, comprising bringing test compounds into contact with each of AdipoR1-deficient cells and AdipoR1 expressing cells to determine whether or not the test compounds cause intracellular influx of extracellular calcium ions and selecting, from the test compounds, compounds not substantially causing intracellular influx of extracellular calcium ions in the AdipoR1-deficient cells and causing intracellular influx of extracellular calcium ions in the AdipoR1 expressing cells as the candidate compounds for adiponectin receptor 1 agonist.

3. A method of screening for a candidate compound for adiponectin receptor 1 agonist, comprising:
(a) bringing test compounds into contact with cells to determine whether or not the test compounds cause intracellular influx of extracellular calcium ions and selecting, from the test compounds, compounds causing intracellular influx of extracellular calcium ions as the candidate compounds for adiponectin receptor 1 agonist; and
(b) bringing the candidate agonist compounds selected in the step (a) into contact with each of AdipoR1-deficient cells and AdipoR1 expressing cells to determine whether or not the candidate agonist compounds cause intracellular influx of extracellular calcium ions and selecting, from the candidate agonist compounds, compounds not substantially causing intracellular influx of extracellular calcium ions in the AdipoR1-deficient cells and causing intracellular influx of extracellular calcium ions in the AdipoR1 expressing cells as agonist compounds for adiponectin receptor 1.

4. The method according to any one of Claims 1 to 3, wherein the cells are skeletal muscle cells.

5. The method according to any one of Claims 1 to 3, wherein the cells are *Xenopus* oocytes injected with AdipoR1 complementary RNA.

6. The method according to any one of Claims 1 to 5, further comprising measuring an intracellular calcium ion concentration.

7. The method according to Claim 6, wherein the intracellular calcium ion concentration is measured in terms of fluorescence intensity by using a calcium ion fluorescent reagent.

8. The method according to any one of Claim 1 to 5, wherein intracellular calcium ion concentration is measured electrophysiologically.

9. The method according to Claim 8, wherein as the cells, cells in which adiponectin receptor 1 has been expressed are used.
